# EUROPEAN PATENT SPECIFICATION

(11) **EP 0 971 923 B1**
(45) Date of publication and mention of the grant of the patent: **06.11.2002**
(21) Application number: 98906239.3
(22) Date of filing: 06.02.1998
(51) Int. Cl.: C07D 473/18, C07D 473/40, C07C 69/003, C07C 69/66, C07C 229/24

(54) **SYNTHESIS OF ACYCLIC NUCLEOSIDE DERIVATIVES**
VERFAHREN ZUR HERSTELLUNG VON AZYKLISCHEN NUKLEOSIDEN
SYNTHESE DE DERIVES DE NUCLEOSIDE ACYCLIQUE

(30) Priority: 10.02.1997 US 798216; 10.02.1997 US 37517 P; 08.08.1997 US 908754
(43) Date of publication of application: 19.01.2000
(73) Proprietor: MEDIVIR AB, S-141 44 Huddinge (SE)
(72) Inventor: LEANNA, M., Robert, Grayslake, IL 60030 (US); HANNICK, Steven, M., Highland Park, IL 60035 (US); RASMUSSEN, Michael, Kenosha, WI 53140 (US); TIEN, Jien-Heh, J., Vernon Hills, IL 60061 (US); BHAGAVATULA, Lakshmi, Vernon Hills, IL 60061 (US); SINGAM, Pulla, Reddy, Des Plaines, IL 60016 (US); GATES, Bradley, D., Mount Prospect, IL 60056 (US); KOLACZKOWSKI, Lawrence, Gurnee, IL 60031 (US); PATEL, Ramesh, R., Chicago, IL 60645 (US); WAYNE, Greg, Vernon Hills, IL 60061 (US); LANNOYE, Greg, Wildwood, IL 60030 (US); ZHANG, Weijiang, Grayslake, IL 60030 (US); TIAN, Zhenping, Grayslake, IL 60030 (US); LUKIN, Kirill, L., Mundelein, IL 60060 (US); NARAYANAN, Bikshandarkor, A., Mundelein, IL 60060 (US); RILEY, David, A., Kenosha, WI 53142 (US); MORTON, Howard, Gurnee, IL 60060 (US); CHANG, Sou-Jen, Praire View, IL 60069 (US)
(74) Representative: Dehmel, Albrecht, Dr.
(86) International application number: US9802439
(87) International publication number: WO98034917

(56) References cited:
- EP-A- 0 343 133
- EP-A- 0 694 547
- WO-A-89/10923
- WO-A-95/22330
- WO-A-97/30051
- WO-A-97/30052
- J-F NAV ET AL: "Synthesis, enzymatic phosphorylation and antiviral activity of acyclic dienyl phosphonate derivatives of guanine" BIORG MED CHEM LETTERS, vol. 6, no. 2, 1996, pages 179-184, XP002065698
- K.OHSAWA ET AL: "Synthesis of optically active nucleoside analogues" CHEM PHARM BUL, vol. 41, no. 11, 1993, pages 1906-1909, XP002065699
- TERAO Y.: 'Synthesis of chiral 3-substituted gamma-lactones and 9-uranosyladenine' CHEM. PHARM BULL vol. 39, no. 3, 1991, pages 823 - 825

## Description

### Technical Field

This invention relates to the field of synthesis of acyclic nucleosides useful against herpes and retroviral infections and novel intermediates.

### Background of the invention

Our copending international applications WO9730051 and WO9730052 disclose diester derivatives of H2G bearing specific combinations of an amino acid ester and a fatty acid ester which are able to provide significantly improved oral bioavailability relative to the parent compound (H2G). These compounds have the formula **I** 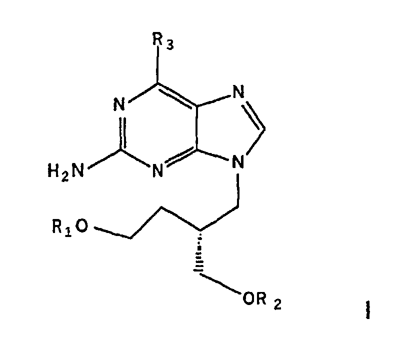 wherein
a) R₁ is -C(O)CH(CH(CH₃)₂)NH₂ or -C(O)CH(CH(CH₃)CH₂CH₃)NH₂ and R₂ is -C(O)C₃-C₂₁ saturated or monounsaturated, optionally substituted alkyl; or
b) R₁ is -C(O)C₃-C₂₁ saturated or monounsaturated, optionally substituted alkyl and R₂ is -C(O)CH(CH(CH₃)₂)NH₂ or -C(O)CH(CH(CH₃)CH₂CH₃)NH₂; and R₃ is OH or H;
or are a pharmaceutically acceptable salt thereof.

Advantageously group R₃ is hydroxy or its tautomer =O so that the base portion is the naturally occuring guanine, for instance in the event that the side chain is cleaved in vivo. Alternatively, R₃ may be hydrogen thus defining the generally more soluble 6-deoxy derivative which can be oxidised in vivo (e.g. by xanthine oxidase) to the guanine form.

The compound of formula **I** may be present in racemic form, that is a mixture of the 2R and 2S isomers. Preferably, however, the compound of formula **I** has at least 70%, preferably at least 90% R form, for example greater than 95%. Most preferably the compound of formula **I** is enantiomerically pure R form.

Preferably the amino acid of group R₁/R₂ is derived from an L-amino acid.

Preferably the fatty acid of group R₁/R₂ has in total an even number of carbon atoms, in particular, decanoyl (C₁₀), lauryl (C₁₂), myristoyl (C₁₄), palmitoyl (C₁₆), stearoyl (C₁₈) or eicosanoyl (C₂₀). Other useful R₁/R₂ groups include butyryl, hexanoyl, octanoyl or behenoyl (C₂₂). Further useful R₁/R₂ groups include those derived from myristoleic, myristelaidic, palmitoleic, palmitelaidic, n6-octadecenoic, oleic, elaidic, gandoic, erucic or brassidic acids. Monounsaturated fatty acid esters typically have the double bond in the trans configuration, preferably in the ω-6, ω-9 or ω-11 position, dependent upon their length. Preferably the R₁/R₂ group is derived from a fatty acid which comprises a C₉ to C₁₇ saturated, or n:9 monounsaturated, alkyl.

The saturated or unsaturated fatty acid of R₁/R₂ may optionally be substituted with up to five similar or different substituents independently selected from the group consisting of such as hydroxy, C₁-C₆ alkyl, C₁-C₆ alkoxy, C₁-C₆ alkoxy C₁-C₆ alkyl, C₁-C₆ alkanoyl, amino, halo, cyano, azido, oxo, mercapto and nitro, and the like.

Most preferred compounds of the formula **I** are those where R₁ is -C(O)CH(CH(CH₃)₂)NH₂ or -C(O)CH(CH(CH₃)CH₂CH₃)NH₂ and R₂ is -C(O)C₉-C₁₇ saturated alkyl.

The term "lower alkyl" as used herein refers to straight or branched chain alkyl radicals containing from 1 to 7 carbon atoms including, but not limited to, methyl, ethyl, n-propyl, iso-propyl, n-butyl, iso-butyl, sec-butyl, t-butyl, n-pentyl, 1-methylbutyl, 2,2-dimethylbutyl, 2-methylpentyl, 2,2-dimethylpropyl, n-hexyl and the like.

The term "alkanoyl" as used herein refers to R₂₀C(O)- wherein R₂₀ is a loweralkyl group.

The term "alkoxy" as used herein refers to R₂₁O- wherein R₂₁ is a loweralkyl group.

The term "alkoxyalkyl" as used herein refers to an alkoxy group appended to a loweralkyl radical.

The term "N-protecting group" or "N-protected" as used herein refers to those groups intended to protect the N-terminus of an amino acid or peptide or to protect an amino group against undesirable reactions during synthetic procedures. Commonly used N-protecting groups are disclosed in Greene, "Protective Groups in Organic Synthesis" (John Wiley & Sons, New York, 1981), which is hereby incorporated by reference. N-protecting groups include acyl groups such as formyl, acetyl, propionyl, pivaloyl, t-butylacetyl, 2-chloroacetyl, 2-bromoacetyl, trifluoracetyl, trichloroacetyl, phthalyl, o-nitrophenoxyacetyl, α-chlorobutyryl, benzoyl, 4-chlorobenzoyl, 4-bromobenzoyl, 4-nitrobenzoyl, and the like; sulfonyl groups such as benzenesulfonyl, p-toluenesulfonyl, and the like, carbamate forming groups such as benzyloxycarbonyl, p-chlorobenzyloxycarbonyl, p-methoxybenzyloxycarbonyl, p-nitrobenzyloxycarbonyl, 2-nitrobenzyloxycarbonyl, p-bromobenzyloxycarbonyl, 3,4-dimethoxybenzyl-oxycarbonyl, 4-methoxybenzyloxycarbonyl, 2-nitro-4,5-dimethoxybenzyloxy-carbonyl, 3,4,5-trimethoxybenzyloxycarbonyl, 1-(p-biphenylyl)-1-methylethoxycarbonyl, α,α-dimethyl-3,5-dimethoxybenzyloxycarbonyl, benzhydryloxycarbonyl, t-butoxycarbonyl, diisopropylmethoxycarbonyl, isopropyloxycarbonyl, ethoxycarbonyl, methoxycarbonyl, allyloxycarbonyl, 2,2,2-trichloroethoxycarbonyl, phenoxycarbonyl, 4-nitrophenoxycarbonyl, fluorenyl-9-methoxycarbonyl, cyclopentyloxycarbonyl, adamantyloxycarbonyl, cyclohexyloxycarbonyl, phenylthiocarbonyl, and the like; alkyl gropus such as benzyl, triphenylmethyl, benzyloxymethyl and the like; and silyl groups such as trimethylsilyl and the like. Favoured N-protecting groups include formyl, acetyl, benzoyl, pivaloyl, t-butylacetyl, phenylsulfonyl, benzyl, t-butoxycarbonyl (BOC) and benzyloxycarbonyl (Cbz).

The term "O-protecting group" or "hydroxy-protecting group" or "-OH protecting group" as used herein refers to a substituent which protects hydroxyl groups against undesirable reactions during synthetic procedures such as those O-protecting groups disclosed in Greene, "Protective Groups In Organic Synthesis," (John Wiley & Sons, New York (1981)). O-protecting groups comprise substituted methyl ethers, for example, methoxymethyl, benzyloxymethyl, 2-methoxyethoxymethyl, 2-(trimethylsilyl)ethoxymethyl, t-butyl, benzyl and triphenylmethyl; tetrahydropyranyl ethers; substituted ethyl ethers, for example, 2,2,2-trichloroethyl; silyl ethers, for example, trimethylsilyl, t-butyldimethylsilyl and t-butyldiphenylsilyl; and esters prepared by reacting the hydroxyl group with a carboxylic acid, for example, acetate, propionate, benzoate and the like.

The term "activated ester derivative" as used herein refers to acid halides such as acid chlorides, and activated esters including, but not limited to, formic and acetic acid derived anhydrides, anhydrides derived from alkoxycarbonyl halides such as isobutyloxycarbonylchloride and the like, N-hydroxysuccinimide derived esters, N-hydroxyphthalimide derived esters, N-hydroxybenzotriazole derived esters, N-hydroxy-5-norbornene-2,3-dicarboxamide derived esters, 2,4,5-trichlorophenyl derived esters, sulfonic acid derived anhydrides (for example, p-toluenesulonic acid derived anhydrides and the like) and the like.

The compounds of SE9600613.5 and SE9600614.3, ie the priority applications for WO97 30051, are prepared by esterification of the H2G parent compound which is prepared, for example, by the synthesis methodology disclosed in European Patent EP 343 133 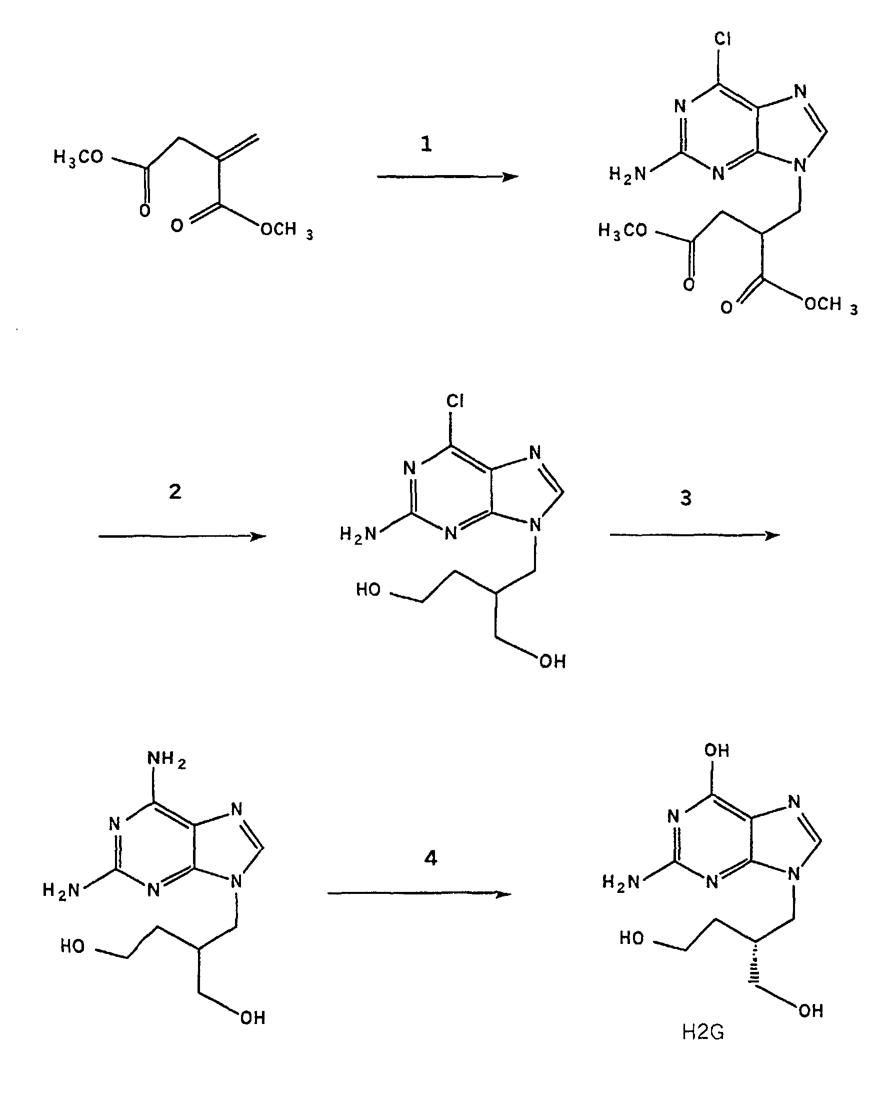 as described below in Schemes A and B.

### A. Direct acylation method

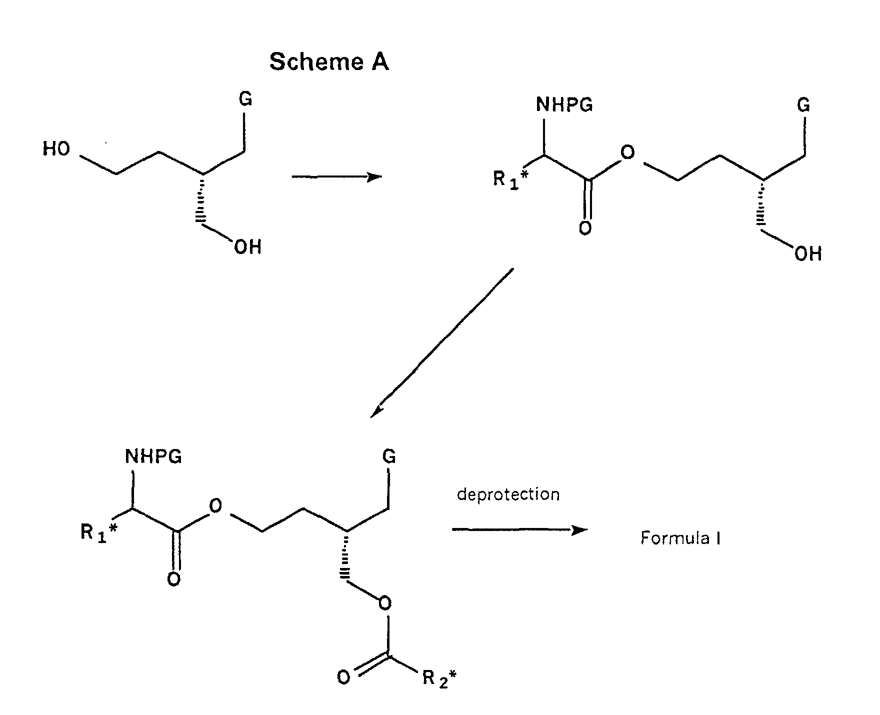

Scheme A depicts the preparation of compounds in which R₁ is derived from the amino acid and R₂ is derived from the fatty acid, but the converse scheme is applicable to compounds where R₁ is derived from the fatty acid and R₂ is derived from the amino acid ester. In the variant specifically depicted in scheme A above, G is guanine or 6-deoxyguanine, PG is an optional N-protecting group or hydrogen, R₁* is the valine or isoleucine side chain and R₂* is the fatty acid chain. H2G is depicted above as a starting material but this of course may be optionally protected at R₃ or the 2 position of the purine with conventional N-protecting groups (not shown). The H2G (derivative) reacts in the first step with an activated R₁ α-amino acid derivative, as further described below, in a solvent such as dimethylformamide or pyridine, to give a monoacylated product. The R₁ α-amino acid may be suitably N-protected with N-BOC or N-CBz or the like. Under controlled conditions, the first acylation can be made to predominantly take place at the side chain 4-hydroxy group on the side chain of H2G. These controlled conditions can be achieved, for example, by manipulating the reagent concentrations or rate of addition, especially of the acylating agent, by lowering the temperature or by the choice of solvent. The reaction can be followed by TLC to monitor the controlled conditions.

After purification, the R₁ monoacylated compounds are further acylated on the side chain 2-CH₂OH group with the appropriate activated fatty acid derivative to give diacylated products using similar procedures as for the first esterification step. The diester products are subsequently subjected to a conventional deprotection treatment using for example trifluoroacetic acid, HCI(aq)/dioxane or hydrogenation in the presence of catalyst to give the desired compound of Formula **I**. The compound may be in salt form depending on the deprotection conditions.

The activated R₁/R₂ acid derivative used in the various acylations may comprise e.g. the acid halide, acid anhydride, activated acid ester or the acid in the presence of coupling reagent, for example dicyclohexylcarbodiimide, where "acid" in each case represents the corresponding R₁/R₂ amino acid or the R₁/R₂ fatty acid. Representative activated acid derivatives include the acid chloride, formic and acetic acid derived mixed anhydrides, anhydrides derived from alkoxycarbonyl halides such as isobutyloxycarbonylchloride and the like, N-hydroxysuccinimide derived esters, N-hydroxyphthalimide derived esters, N-hydroxy-5-norbomene-2,3-dicarboxamide derived esters, 2,4,5-trichlorophenol derived esters, sulfonic acid derived anhydrides (for example, p-toluenesulonic acid derived anhydrides and the like) and the like.

### B. Via protection of the side chain 4-hydroxy group:

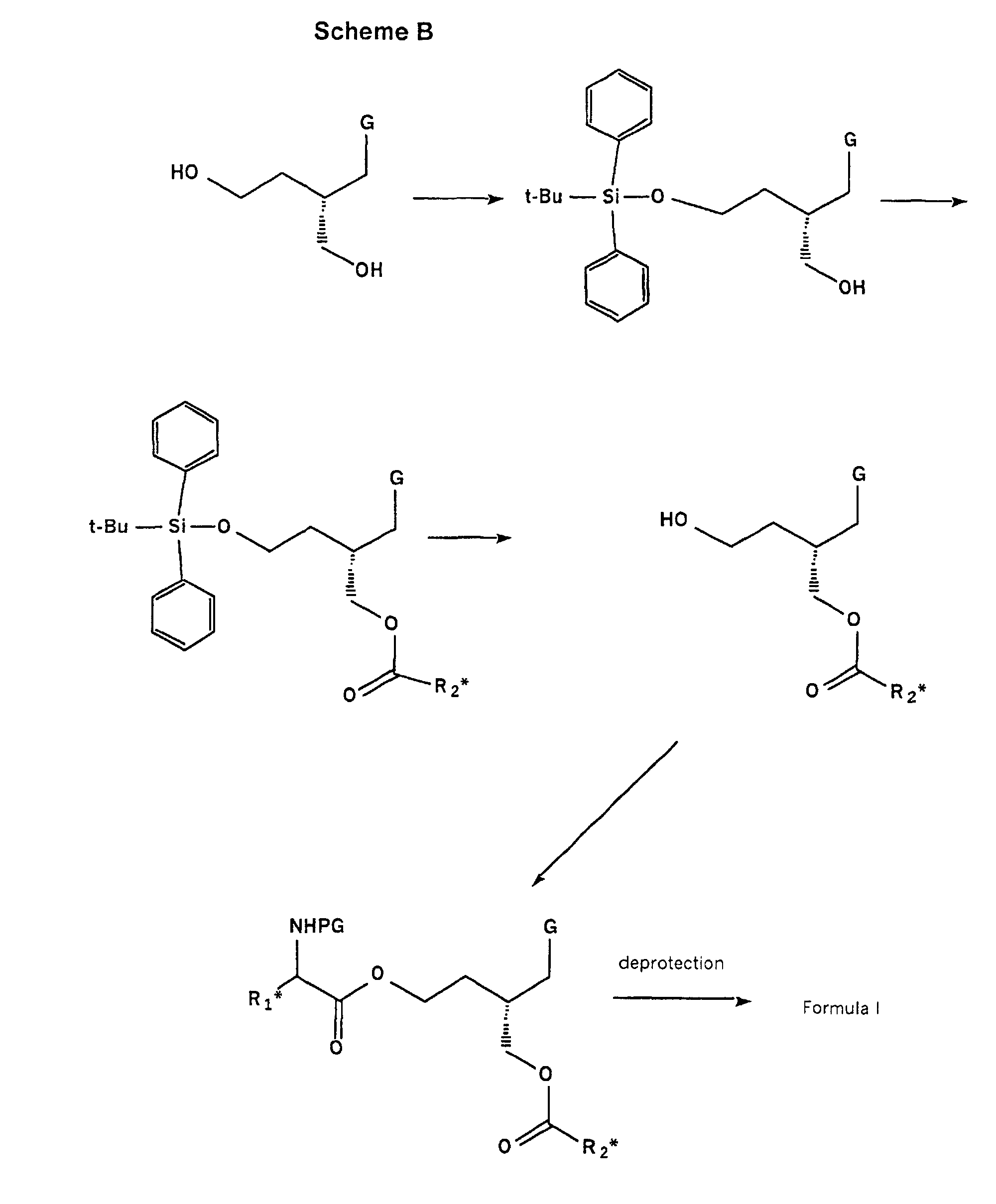

wherein G, PG, R₁* and R₂* are as described for scheme A.

Scheme B has been exemplified with reference to the preparation of a compound where R₁ is derived from an amino acid and R₂ is derived from the fatty acid ester, but a converse scheme will be applicable to compounds where R₂ is derived from the amino acid and R₁ is derived from the fatty acid. This scheme relies on regioselective protection of the H2G side chain 4-hydroxy group with a bulky protecting group. In scheme B above this is depicted as t-butyldiphenylsilyl, but other regioselective protecting groups such as trityl, 9-(9-phenyl)xanthenyl, 1,1-bis(4-methylphenyl)-1'-pyrenylmethyl may also be appropriate. The resulting product is acylated at the side chain 2-hydroxymethyl group using analogous reagents and procedures as described in scheme A above, but wherein the activated acid derivative is the R₂ fatty acid, for example, myristic, stearic, oleic, elaidic acid chloride and the like. The thus monoacylated compounds are subjected to appropriate deprotection treatment to remove the side chain 4-hydroxy protecting group which can be done in a highly selective manner with such reagents, depending on the regioselective protecting group, as HF/pyridine and the like and manipulation of the reaction conditions, viz reagent concentration, speed of addition, temperature and solvent etc, as elaborated above. The then free side chain 4-hydroxy group is acylated with the activated α-amino acid in a similar way as described in scheme A above.

Additional techniques for introducing the amino acid ester of R₁/R₂, for instance in schemes A, B, C, D or E herein include the 2-oxa-4-aza-cycloalkane-1,3-dione method described in International patent application No. WO 94/29311.

Additional techniques for introducing the fatty acid ester of R₁/R₂, for instance in schemes A, B, C, D or E herein include the enzymatic route described in Preparative Biotransformations 1.11.8 (Ed S M Roberts, J Wiley and Son, NY, 1995) with a lipase such as SP 435 immobilized Candida antarcticus (Novo Nordisk), porcine pancreatic lipase or Candida rugosa lipase. Enzymatic acylation is especially convenient where it is desired to avoid N-protection and deprotection steps on the other acyl group or the purine 2-amine.

An alternative route to compounds of Formula **I** in which R₃ is hydrogen is to 6-activate the correponding guanine compound of Formula **I** (wherein the amino acid ester moiety of R₁/R₂ is optionally protected with conventional N-protecting groups such as BOC) with an activating group such as halo. The thus activated 6-purine is subsequently reduced to purine, for instance with a palladium catalyst and deprotected to the desired 6-deoxy H2G di-ester.

Schemes A and B above employ selective acylation to stepwise add the amino acid and fatty acid esters. An alternative process claimed in WO9730052 for the preparation of the compounds of formula **I** starts with a diacylated H2G derivative, wherein both the acyl groups are the same, and employs selective removal of one of the acyl groups to obtain a monoacyl intermediate which is then acylated with the second, differing, acyl group in the same manner as Schemes A and B above.

### Brief Description of the Invention

The present invention provides alternative synthetic routes to the compounds of Formula I and novel intermediates for use therein. A first aspect of the invention provides compounds as set out in independent claims 1, 9, 20, 25, 31, 39, and 41 as well as processes to prepare such compounds, which processes are set out in independent claims 6, 12, 14, 30, and 38.

An exemplary process employing various aspects of the invention is the synthesis of compounds of the formula **I** wherein R₃ is -OH as shown in Scheme C.

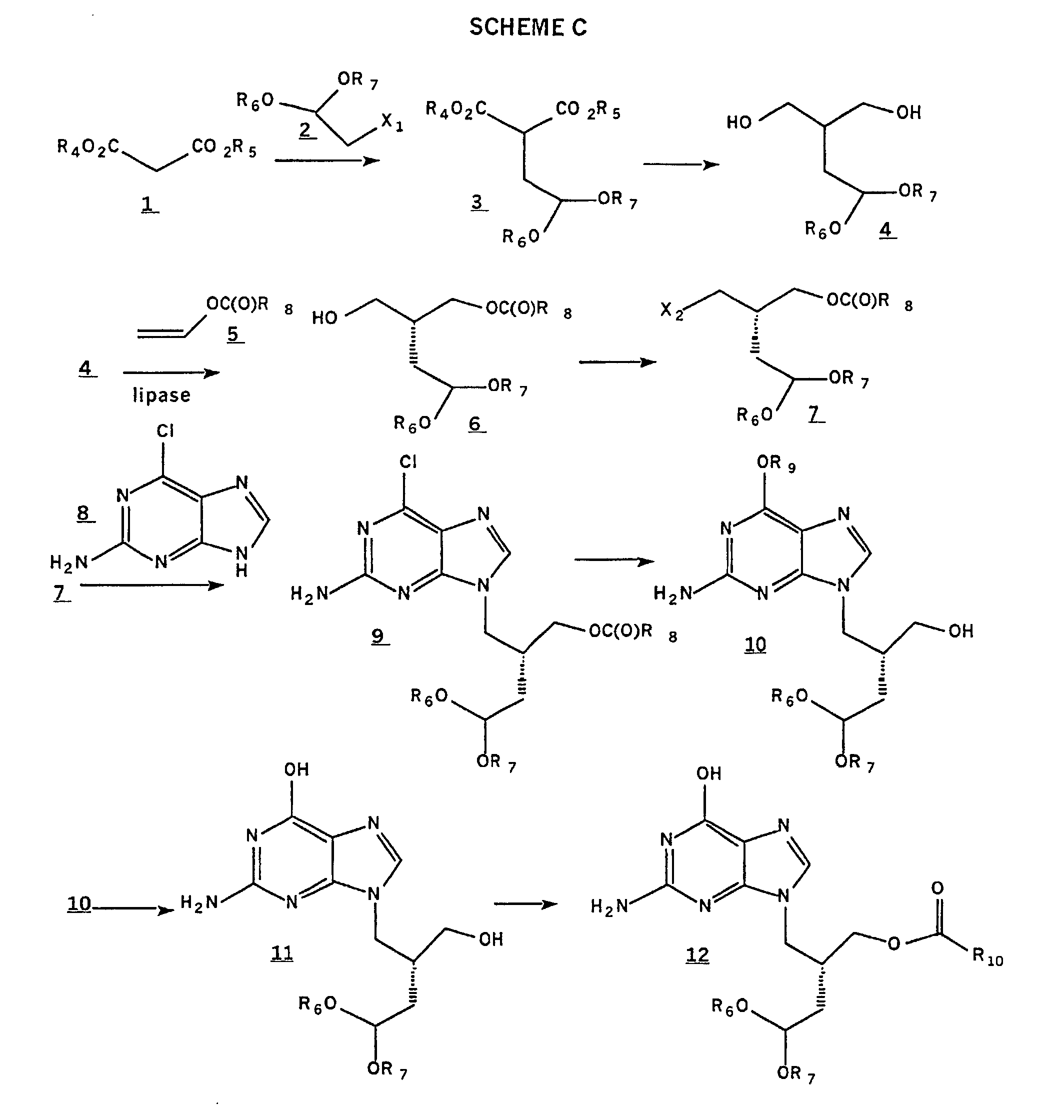

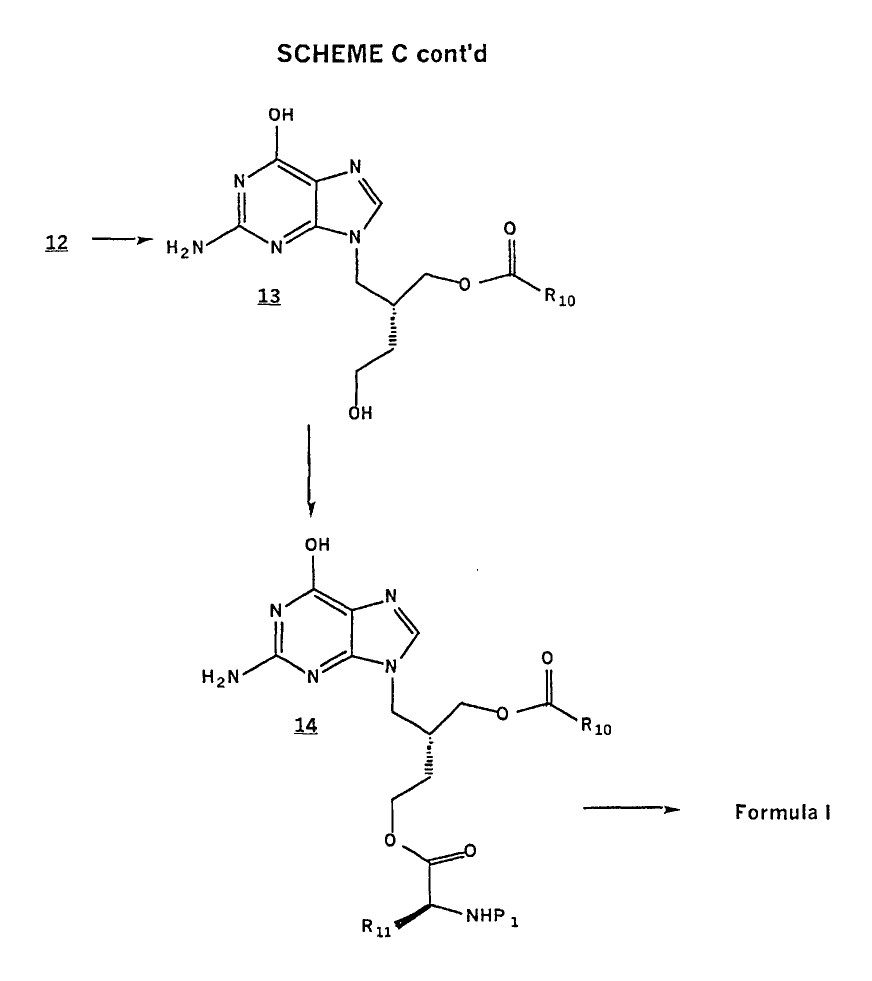

Referring to Scheme C, malonate **1** (R₄ and R₅ are lower alkyl or benzyl or the like) is alkylated by reaction with from about 0.5 to about 2.0 molar equivalents of acetal **2** (R₆ and R₇ are lower alkyl or benzyl and the like or R₆ and R₇ taken together are -CH₂CH₂- or -CH₂CH₂CH₂- or -CH₂CH₂CH₂CH₂-and X₁ is a leaving group (for example, Cl, Br or I, or a sulfonate such as methanesulfonate, triflate, p-toluenesulfonate, benzenesulfonate and the like)) in the presence of from about 0.5 to about 2.0 molar equivalents of a base (for example, potassium t-butoxide or sodium ethoxide or NaH or KH and the like) in an inert solvent (for example, DMF or THF or dioxane or dioxolane or N-methylpyrrolidone and the like) at a temperature of from about -40°C to about 190°C to provide alkylated malonate **3**. Alkylated malonate **3** can be purified by distillation or by first treating the crude alkylated malonate with dilute aqueous base (for example, 7% aqueous KOH), followed by removal of volatile impurities by distillation.

Reduction of **3** with from about 0.5 to about 4.0 molar equivalents of an ester to alcohol reducing agent (for example, LiBH₄ or Ca(BH₄)₂ or NaBH₄ or LiAIH₄ and the like) in an inert solvent (for example, THF or methyl t-butyl ether or t-BuOH and the like) at a temperature of from about -20°C to about 100°C provides diol **4**. Enzymatic esterification of **4** by reaction with from about 1.0 to about 20.0 molar equivalents of a vinyl ester **5** (R₈ is C₁-C₂₁ saturated or monounsaturated, optionally substituted alkyl) in the presence of a lipase (for example, Lipase PS-30 or Lipase PPL or Lipase CCL and the like) or a phospholipase (for example phospholipase D and the like) provides the desired stereoisomer of ester **6**. This reaction can be carried out in the absence of solvent or in the presence of an inert solvent (for example, methyl t-butyl ether or toluene or hexane and the like). The reaction is carried out at a temperature of from about -20°C to about 80°C.

The alcohol substituent of **6** is converted to a leaving group (for example, a halogen or a sulfonate) by reaction with a halogenating agent (for example NBS/P(Ph)₃ or NCS/P(Ph)₃ or POCI₃ or NCS/P(Ph)₃/Nal in acetone and like) in an inert solvent (for example, methylene chloride or toluene or ethylacetate and the like) or by reaction with from about 0.8 molar equivalents to about 2.0 molar equivalents of a sulfonyl halide (for example, benzenesulfonylchloride, toluenesulfonylchloride or methane sulfonylchloride and the like) in the presence of from about 1.0 to about 4.0 molar equivalents of a base (for example, triethylamine or potassium carbonate or pyridine or dimethylaminopyridine or ethyldiisopropylamine and the like) in an inert solvent (for example methylene chloride or toluene or ethylacetate or pyridine or methyl t-butyl ether and the like) at a temperature of from about -25°C to about 100°C to provide ester **7** according to the invention (X₂ is a halogen or sulfonate leaving group).

Reaction of **7** with from about 0.9 to about 2.0 molar equivalents of 2-amino-6-chloropurine **8** in the presence of from about 1.0 to about 6.0 molar equivalents of a base (for example, potassium carbonate or NaH or KH or NaOH or KOH or lithium diisopropylamide or LiN(Si(CH₃)₃)₂ and the like) in an inert solvent (for example, DMF or THF or acetonitrile or N-methylpyrrolidone or ethanol or DMSO and the like) at a temperature of from about -25 °C to about 140°C provides substituted purine **9**.

Alternatively Mitsunobu coupling (for example P(Ph)₃/diethyl azidocarboxylate) of alcohol **6** with 2-amino-6-chloropurine **8** provides **9**.

Reaction of **9** with from about 2.0 to about 20 molar equivalents of an alcohol R₉OH (R₉ is an alcohol protecting group such as benzyl or diphenylmethyl and the like) in the presence of from about 1.0 to about 6.0 molar equivalents of a base (for example, potassium t-butoxide or potassium carbonate or NaH or KH or lithium diisopropylamide and the like) in an inert solvent (for example, THF or DMF and the like) at a temperature of from about -25°C to about 150°C provides alcohol **10** according to the invention.

Removal of the alcohol protecting group R₉ of **10** (for example, by catalytic hydrogenation in an inert solvent such as ethanol or benzyl alcohol or methanol or THF and the like in the presence of an hydrogenation catalyst such as Pd/C or Pd(OH)₂ and the like) provides substituted guanine **11**.

Esterification of **11** by reaction with a) from about 0.8 to about 2.0 molar equivalents of R₁₀COOH and a coupling agent (for example DCC/DMAP) and the like in an inert solvent (for example THF or DMF and the like) or b) from about 0.8 to about 2.0 molar equivalents of an activated derivative of R₁₀COOH (for example, the acid chloride or N-hydroxysuccinimide ester or R₁₀C(O)OS(O)₂R₃₀ (R₃₀ is loweralkyl, phenyl or toluyl) or R₁₀C(O)OC(O)R₁₀ or R₁₀C(O)OC(O)R₁₀ₐ (R₁₀ₐ is loweralkyl and the like) in the presence of from about 0 to about 3.0 molar equivalents of a base (for example, pyridine or dimethylaminopyridine or triethylamine or ethyldiisopropylamine or N-methylmorpholine or DBU or potassium carbonate and the like) in an inert solvent (for example, methylene chloride or THF or pyridine or acetonitrile or DMF and the like) at a temperature of from about -25°C to about 100°C provides ester **12**, which ester represents one aspect of the present invention. R₁₀ is C₃-C₂₁ saturated or monounsaturated, optionally substituted alkyl.

The acetal substituent of **12** is deprotected and the resulting aldehyde is reduced by first reacting **12** with from about 0.1 to about 10.0 molar equivalents of an acid (for example, triflic acid or HCI or formic acid or acetic acid/formic acid or sulfuric acid and the like) in an inert solvent (for example, THF/H₂O or methylene chloride/H₂O or ethylacetate/H₂O or ethanol/H₂O or methanol/H₂O or water and the like) at a temperature of from about -25°C to about 100°C. To the crude reaction mixture is added from about 0.1 to about 10.0 molar equivalents of a base (for example, sodium bicarbonate or potassium carbonate or triethylamine or pyridine or KOH and the like), (optionally, additional inert solvent (for example, THF and or methylene chloride or ethylacetate or methyl t-butyl ether or isopropoanol and the like) is added) and from about 0.3 to about 5.0 molar equivalents of an aldehyde reducing agent (for example, sodium borohydride or RaNi/H₂ or borane t-butylamine complex and the like) at a temperature of from about -25 °C to about 100°C to provide alcohol **13**. The optical purity of compound **13** can be enhanced by reaction with optically active organic sulfonic acids such as (S)(+)-camphorsulfonic acid and the like. A preferred sulfonic acid for this purpose is (S)-(+)-camphorsulfonic acid.

Alternatively, the acetal substituent of **12** can be hydrolyzed by reaction in an inert solvent with an acid resin (for example, Amberlyst 15 resin, Nafion NR50 resin, Dowex 50WX4-200R resin or Amberlite 120 resin and the like) to provide the corresponding aldehyde. The aldehyde can be isolated prior to reduction to the alcohol **13** as described above or the crude aldehyde can be reduced directly *in situ.*

Reaction of **13** with from about 0.8 to about 3.0 molar equivalents of N-protected amino acid P₁NHCH(R₁₁)COOH or an activated derivative thereof (P₁ is an N-protecting group and R₁₁ is isopropyl or isobutyl) in an inert solvent (for example, THF or dioxane or dioxolane or DMF or methylene chloride and the like) at a temperature of from about 25°C to about 100°C provides alcohol **14**.

N-deprotection of **14** provides the compound of formula **I** wherein R₃ is -OH.

Alternatively, compound **13** can be reacted with the symmetrical anhydride derived from P₁NHCH(R₁₁)COOH (i.e., P₁NHCH(R₁₁)C(O)O-C(O)CH(R₁₁)NHP₁) to provide **14**. The anhydride can be prepared *in situ* or can be separately prepared prior to reaction with **13**.

Alternatively, **11** can be prepared by hydrolysis of the ester of **9** to an alcohol (for example, by reaction with K₂CO₃ in MeOH/H₂O and the like), followed by direct conversion of the chloro group to an -OH (for example, by reaction with an inorganic base such as KOH or NaOH and the like in H₂O with heating and the like).

In another alternative method, **11** can be prepared directly by hydrolysis of the chloro-ester **9** (for example, by reaction with an inorganic base such as KOH or NaOH and the like in H₂O with heating and the like).

In another alternative method, **11** can be prepared from **9** (or from the hydroxy compound resulting from the hydrolysis of the ester in **9**) by reaction with an inorganic base (for example, NaOH, LiOH, KOH and the like, preferably, NaOH) and trimethylamine in an aqueous solvent.

In yet another alternative method, **11** can be prepared directly by hydrolysis of the chloro-ester **9** (for example, by reaction with 1-3 equivalents of a base such as sodium methoxide (and the like) in the presence of mercaptoethanol in a mixed solvent of water and methanol or dioxane (and the like) at a temperature of from about 20°C to about relfux and the like).

In yet another alternative method, 13 can be prepared by reaction of **9** (wherein R₈=R₁₀)with formic acid, optionally with heating, followed by reduction of the aldehyde to give **13**.

Other aspects of the invention are embodied in an alternative process for the preparation of compounds of Formula **I** wherein R₃ is -OH as shown in Scheme D.

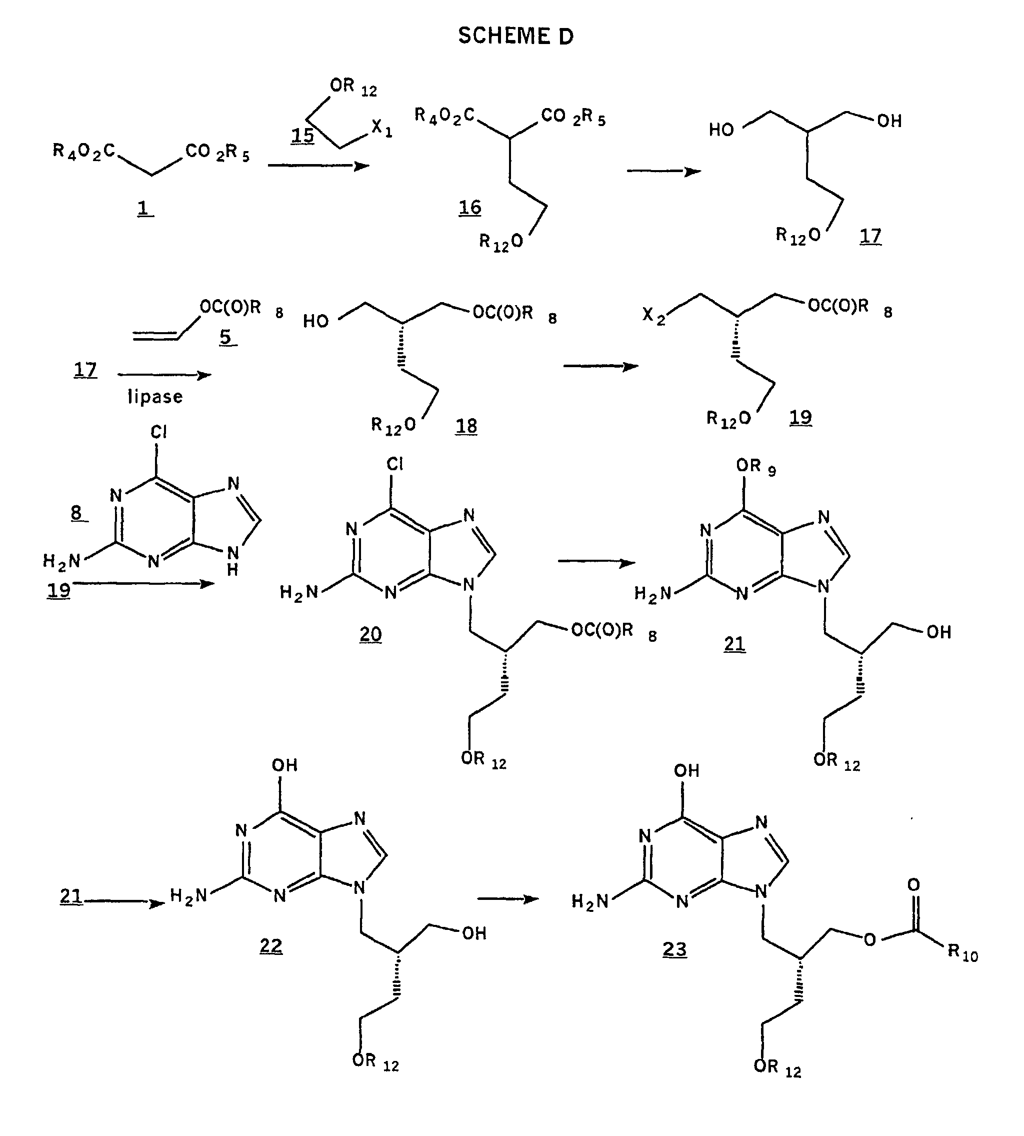

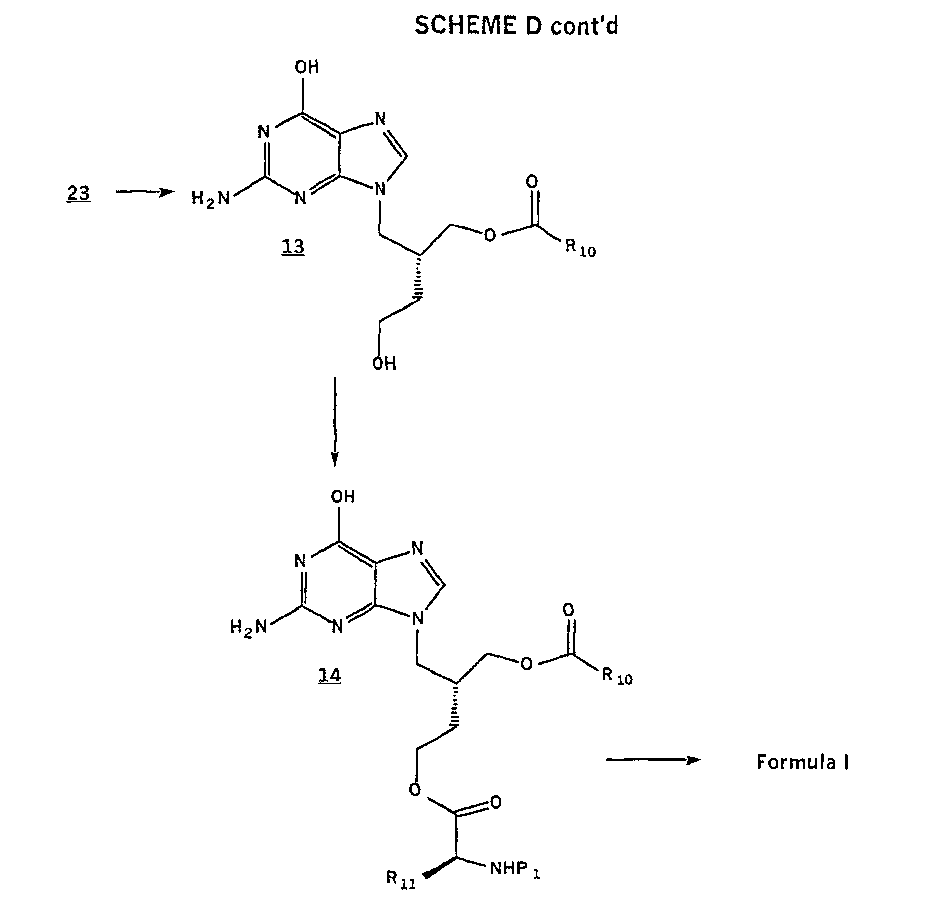

Malonate **1** (R₄ and R₅ are lower alkyl or benzyl and the like) is alkylated with from about 0.5 to about 2.0 molar equivalents of ether **15** wherein X₁ is a leaving group (for example Cl, Br or I, or a sulfonate such as methane sulfonate, triflate, p-toluenesulfonate, benzenesulfonate and the like) and R₁₂ is -CH(Ph)₂, -C(Ph)₃ or -Si(t-Bu)(Me)₂ and the like (Ph = phenyl) in the presence of from about 0.5 to about 2.0 molar equivalents of a base (for example potassium t-butoxide or sodium ethoxide or NaH or KH and the like) in an inert solvent (for example DMF or THF or dioxane or dioxolane or N-methyl pyrrolidinone and the like) at a temperature of from about -40°C to about 190°C to provide alkylated malonate **16.**

Reduction of **16** with from about 0.5 to about 4.0 molar equivalents of an ester to alcohol reducing agent (for example LiBH₄ or Ca(BH₄)₂ or NaBH₄ or LiAIH₄ and the like) in an inert solvent (for example, THF or methyl t-butyl ether or ethanol or t-butanol and the like) at a temperature of from about -20°C to about 100°C provides diol **17.** Enzymatic esterification of **17** by reaction with from about 1.0 to about 20.0 molar equivalents of a vinyl ester **5** (R₈ is C₁-C₂₁ saturated or monounsaturated, optionally substituted alkyl) in the presence of a lipase (for example, Lipase PS-30 or Lipase PPL or Lipase CCL and the like) or a phospholipase (for example phospholipase D and the like) provides the desired stereoisomer of ester **18**. The reaction can be carried out in the absence of solvent or in the presence of an inert solvent (for example methyl t-butyl ether or toluene or hexane or the like). The reaction is carried out at a temperature of from about -20°C to about 80°C.

The alcohol substituent of **18** is converted to a leaving group (for example a halogen or sulfonate) by reaction with a halogenating agent (for example NBS/P(Ph)₃ or NCS/P(Ph)₃ or POCI₃ or NCS/P(Ph)₃/Nal in acetone and the like) in an inert solvent (for example methylene chloride or toluene or ethylacetate and the like) or by reaction with from about 0.8 molar equivalents to about 2.0 molar equivalents of a sulfonyl halide (for example benzenesulfonylchloride, toluenesulfonylchloride or methane sulfonylchloride and the like) in the presence of from about 1.0 to about 4.0 molar equivalents of a base (for example triethylamine or potassium carbonate or pyridine and the like) in an inert solvent (for example, methylene chloride or toluene or ethyl acetate or methyl t-butyl ether and the like) at a temperature of from about -25°C to about 100°C to provide ester **19** (X₂ is a halogen or sulfonate leaving group).

Reaction of **19** with from about 0.9 to about 2.0 molar equivalents of 2-amino-4-chloropurine **8** in the presence of from about 1.0 to about 6.0 molar equivalents of a base (for example potassium carbonate or LiH or NaH or KH or NaOH or KOH or lithium diisopropylamide or LiN(Si(CH₃)₃)₂ and the like) in an inert solvent (for example DMF or THF or acetonitrile or N-methylpyrrolidone or ethanol and the like) at a temperature of from about -25°C to about 140°C provides substituted purine **20**. Alternatively, Mitsunobu coupling (for example, P(PH)₃/diethyl azidocarboxylate) of alcohol **18** with 2-amino-4-chloropurine **8** provides **20**.

Reaction of **20** with from about 2.0 to about 20.0 molar equivalents of an alcohol R₉OH (R₉ is an alcohol protecting group such as benzyl and the like) in the presence of from about 1.0 to about 6.0 molar equivalents of a base (for example, potassium t-butoxide or potassium carbonate or NaH or KH or lithium diisopropylamide and the like in an inert solvent (for example, THF or DMF and the like) at a temperature of from about -25°C to about 150°C provides alcohol **21** covered by one aspect of the present invention.

Removal of the alcohol protecting group R₉ of **21** (for example by catalytic hydrogenation in an inert solvent such as ethanol or benzyl alcohol or methanol or THF and the like in the presence of an hydrogenation catalyst such as Pd/C or Pd(OH)₂ and the like) provides substituted guanine **22**, which can be esterified as described in Scheme C (i.e., **11** to **12**) to provide **23**, the latter compounds being encompassed by the present invention.

The ether substitutent of **23** is deprotected by reaction with a) a reducing agent (for example, HCO₂H and Pd/C and the like) wherein R₁₂ is -CH(Ph)₂ or -C(Ph)₃, or b) a desilylating agent (for example Bu₄NF and the like) wherein R₁₂ is -Si(t-Bu)(Me)₂ and the like to provide **13**.

Alcohol **13** can be converted to **I** as outlined in Scheme C.

Alternatively, **22** can be prepared by hydrolysis of the ester of **20** to an alcohol (for example, by reaction with K₂CO₃ in MeOH/H₂O and the like), followed by direct conversion of the chloro group to an -OH (for example, by reaction with KOH in H₂O with heating and the like).

In another alternative method, **22** can be prepared directly by hydrolysis of the chloro-ester **20** (for example, by reaction with KOH in H₂O with heating and the like).

In another alternative method, **22** can be prepared from **20** (or from the hydroxy compound resulting from the hydrolysis of the ester in **20**) by reaction with an inorganic base (for example, NaOH, LiOH, KOH and the like, preferably, NaOH) and trimethylamine in an aqueous solvent.

In yet another alternative method, **22** can be prepared directly by hydrolysis of the chloro-ester **20** (for example, by reaction with 1-3 equivalents of a base such as sodium methoxide (and the like) in the presence of mercaptoethanol in a mixed solvent of water and methanol or dioxane (and the like) at a temperature of from about 20°C to about relfux and the like).

In yet another alternative method, **23**, another compound of the present invention, can be prepared by reaction of **20** (wherein R₈=R₁₀) with formic acid, optionally with heating, followed by reduction of the aldehyde to give **23**.

An additional alternative involves enzymatic esterification of alcohol **4** or **17** with the vinyl ester CH₂=CH-OC(O)R₁₀ (i.e., R₈ = R₁₀ in Schemes C and D) to directly incorporate into **6** or **18** the desired carboxylic acid ester of the final product **I**. This allows the elimination of the ester hydrolysis and reesterification involved in going from **9** to **12** or from **20** to **23**.

The processes of Schemes C and D are characterized by the fact that each of the hydroxyl groups of the acyclic side chain is differentiated by the use of different hydroxy protecting groups or precursor groups. This allows the selective acylation of each of the hydroxy groups with either an amino acid or a fatty acid group.

Schemes C and D have been illustrated and described with reference to embodiments of the invention wherein R₁ is derived from an amino acid and R₂ is derived from a fatty acid. However, it will be apparent that respective converse schemes will apply to compounds where R₁ is derived from a fatty acid and R₂ is derived from an amino acid.

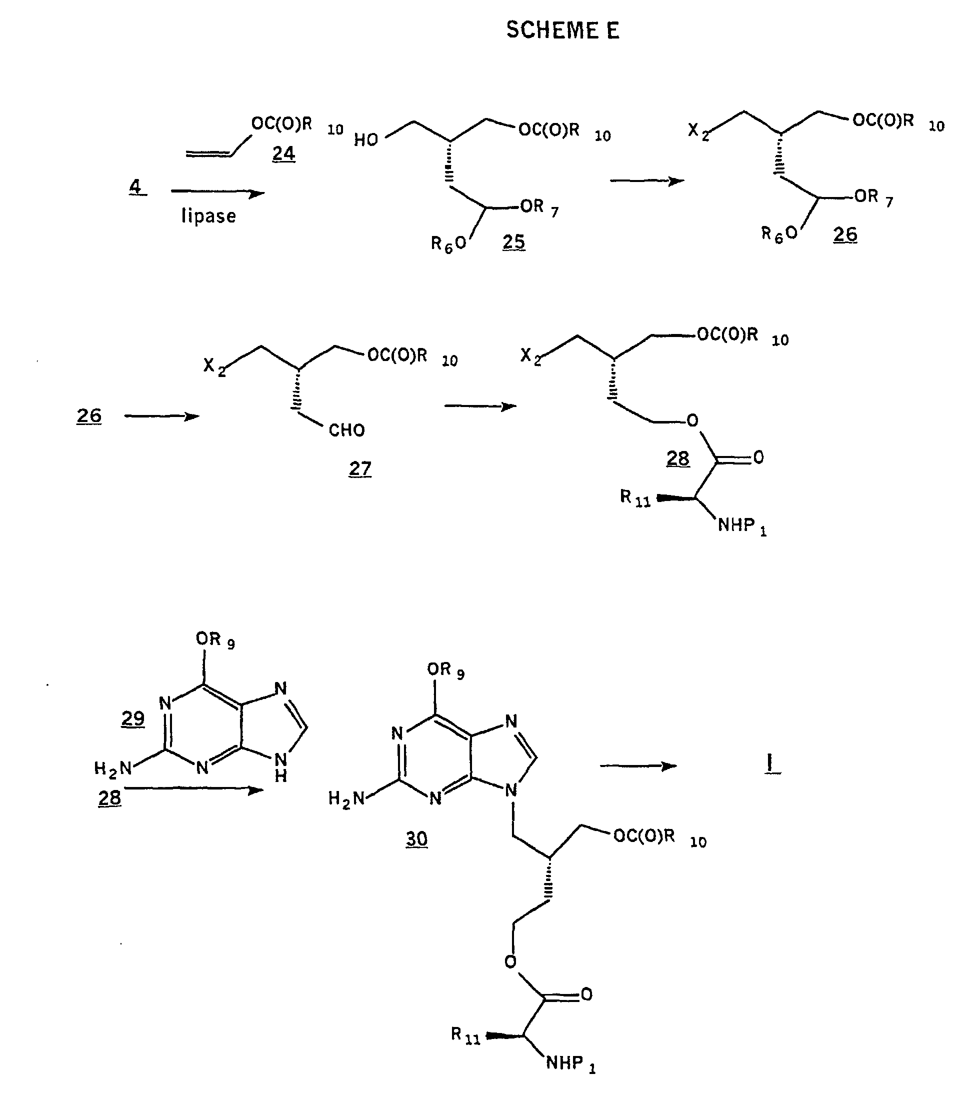

Yet another method for preparing compounds of Formula **I** is shown in Scheme E. Enzymatic esterification of **4** (see Scheme C) by reaction with from about 1.0 to about 20.0 molar equivalents of a vinyl ester **24** (R₁₀ is C₃-C₂₁ saturated or monounsaturated, optionally substituted alkyl) in the presence of a lipase (for example, Lipase PS-30 or Lipase PPL or Lipase CCL and the like) or a phospholipase (for example phospholipase D and the like) provides the desired stereoisomer of ester **25**. This reaction can be carried out in the absence of solvent or in the presence of an inert solvent (for example, methyl t-butyl ether or toluene or hexane and the like). The reaction is carried out at a temperature of from about -20°C to about 80°C.

The alcohol substituent of **25** is converted to a leaving group (for example, a halogen or a sulfonate) by reaction with a halogenating agent (for example NBS/P(Ph)₃ or NCS/P(Ph)₃ or POCI₃ or NCS/P(Ph)₃/Nal in acetone and like) in an inert solvent (for example, methylene chloride or toluene or ethylacetate and the like) or by reaction with from about 0.8 molar equivalents to about 2.0 molar equivalents of a sulfonyl halide (for example, benzenesulfonylchloride, toluenesulfonylchloride or methane sulfonylchloride and the like) in the presence of from about 1.0 to about 4.0 molar equivalents of a base (for example, triethylamine or potassium carbonate or pyridine or dimethylaminopyridine or ethyldiisopropylamine and the like) in an inert solvent (for example methylene chloride or toluene or ethylacetate or pyridine or methyl t-butyl ether and the like) at a temperature of from about -25°C to about 100°C to provide ester **26** (X₂ is a halogen or sulfonate leaving group).

The acetal substituent of **26** is hydrolyzed to the aldehyde **27** according to the invention by reacting **26** with an acid (for example, trifluoroacetic acid, triflic acid or HCI or acetic acid or sulfuric acid and the like) in an inert solvent (for example, THF/H₂O or methylene chloride/H₂O or ethylacetate/H₂O or ethanol/H₂O or methanol/H₂O or water and the like) at a temperature of from about -25 °C to about 100°C. To the aldehyde **27** in an inert solvent (for example, THF and or methylene chloride or ethylacetate or methyl t-butyl ether or isopropoanol and the like) is added an aldehyde to alcohol reducing agent (for example, sodium borohydride or RaNi/H₂ or borane t-butylamine complex and the like) at a temperature of from about -25 °C to about 100°C to provide the corresponding alcohol.

Reaction of the resulting alcohol with from about 0.8 to about 3.0 molar equivalents of N-protected amino acid P₁NHCH(R₁₁)COOH or an activated derivative thereof (P₁ is an N-protecting group and R₁₁ is isopropyl or isobutyl) in an inert solvent (for example, THF or dioxane or dioxolane or DMF or methylene chloride and the like) at a temperature of from about 25°C to about 100°C provides diester **28**.

Alternatively the alcohol can be reacted with the symmetrical anhydride derived from P₁NHCH(R₁₁)COOH (i.e., P₁NHCH(R₁₁)C(O)O-C(O)CH(R₁₁)NHP₁) to provide **28**, which is a further compound of the present invention.

Reaction of **28** with purine **29** in the presence of a base (for example K₂CO₃ and the like) in an inert solvent (for example, DMF and the like) provides **30**. Purine **29** is prepared from 6-chloro-2-amino purine by reaction with R₉OH in an inert solvent (for example, toluene or THF and the like) in the presence of a base (for example, NaH or KH and the like). Substituted purine **30** is deprotected to provide the compound of Formula **I**.

### Detailed Description of the Invention

The invention will now be illustrated by way of example only with reference to the following non-limiting Examples.

### EXAMPLE 1

### Preparation of (R)-9-[4-Hydroxy-2-(stearoyloxymethyl)butyl]guanine

### a) Preparation of ethyl 4,4-diethoxy-2-ethoxycarbonyl-butyrate

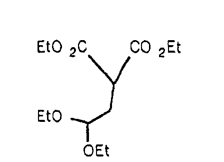

Potassium tert-butoxide (141.8g, 1.11 equiv.) was dissolved in dry DMF (1 L). Diethyl malonate (266 mL, 1.54 equiv.) was added over 5 minutes. Bromoacetaldehyde diethylacetal (172 mL, 1.14 mole) was added over 5 minutes. The mixture was heated to 120° C (internal temperature), and stirred at 120° C for 5 hours. The mixture was allowed to cool to room temperature, poured into water (5 L), and extracted with methyl tert-butyl ether (MTBE, 3 x 600 mL). The organic solution was dried over MgSO₄, filtered, concentrated, and distilled (0.5 mm, 95-140° C) to yield the desired diester (244 g, 78%) as a colorless oil.
¹H NMR (CDCI₃) δ 1.19 (t, 6H), 1.28 (t, 6H), 2.22 (dd, 2H), 3.49 (m, 2H), 3.51 (t, 1H), 3.65 (m, 2H) 4.20 (qd, 4H), 4.54 (t, 1H).

### b) Preparation of 4,4-diethoxy-2-(hydroxymethyl)-butanol

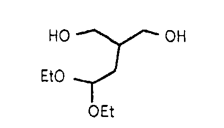

LiBH₄ (purchased solution, 2M in THF, 22.5 mL) and the product of Example 1- step a) (5 g in 15 mL of THF, 18.1 mmol) were combined and warmed to 60° C and stirred at 60° C for 4 hours. The reaction mixture was allowed to cool to room temperature and the reaction vessel was placed in a cool water bath. Then triethanolamine (5.97 mL, 1 equiv.) was added at such a rate that the temperature of the reaction mixture was maintained between 20-25 °C. Brine (17.5 mL) was added at a rate such that gas evolution was controlled and the mixture was stirred for 45 minutes at room temperature. The layers were separated, the organic layer was washed with brine (2 x 15 mL). The combined brine washes were extracted with MTBE (methyl tert-butyl ether, 3 x 20 mL). The combined organic extracts were evaporated and the residue was dissolved in MTBE (50 mL) and washed with brine (25 mL). The brine layer was back-extracted with MTBE (3 x 25 mL). The combined organic extracts were dried over Na₂SO₄, filtered, and concentrated to yield the desired diol (3.36g, 15.5 mmol, 97%) as a colorless oil.
¹H NMR (CDCl₃) δ 1.22 (t, 6H), 1.73 (dd, 2H), 1.92 (m, 1H), 2.67 (bs, 2H), 3.52 (m, 2H), 3.69 (m, 2H), 3.72 (m, 4H), 4.62 (t, 1H).

### c) Preparation of (2R)-2-acetoxymethyl-4,4-diethoxy-butanol

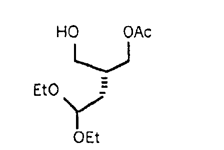

Into a 10 ml 1 neck round bottom flask was charged the product of Example 1 step b) (3.84 g, 20 mmol), followed by addition of vinyl acetate (2.6 g, 30 mmol) and finally Lipase PS 30 (69 mg, purchased from Amano, Lombard, Illinois). The mixture was allowed to stir at ambient temperature for 16 hours. Progress of the reaction was closely monitored by TLC (2/1 hexane - EtOAc; stained with Ce₂(SO₄)₃ and charred on hot plate; r.f. of diol is 0.1, monoacetate is 0.3, bis acetate is 0.75). The reaction mixture was diluted with CH₂Cl₂ and filtered through a 5 micron filter. The filter was washed with additional CH₂Cl₂. The filtrate was then concentrated in vacuo to afford the desired product.

### d) Preparation of (2S)-2-acetoxymethyl-4,4-diethoxybutyl toluenesulfonate e)

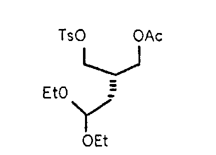

Into a 100 mL 1-neck round bottom flask, equipped with a magnetic stir bar and septum under N₂ was charged the crude product of Example 1 step c) (4.62 g, 19 mmol), dry CH₂Cl₂ (20 mL) and Et₃N (5.62 mL, 40 mmol). To this solution was added tosyl chloride (4.76 g, 25 mmol). The resulting mixture was stirred at ambient temperature for 4 hours. Charged H₂O (0.27 g, 15 mmol) and stirred vigorously for 4 hours. The reaction mixture was diluted with 80 mL EtOAc and 50 mL H₂O and the aqueous layer was separated. To the organic layer was added 75 ml of a 5 % aq. solution of KH₂PO₄. After mixing and separation of the layers, the aqueous layer was removed. The organic layer was washed with 50 mL of saturated NaHCO₃ solution, dried over Na₂SO₄, filtered and concentrated in vacuo to a constant weight of 7.40 g of the desired product.
¹H NMR (CDCI₃) δ 1.17 (t, 6H); 1.62 (m, 2H); 1.94 (s, 3H); 2.19 (m, 1H); 2.45 (s, 3H); 3.42 (m, 2H); 3.6 (m, 2H); 4.03 (m, 4H); 4.51 (t, 1H); 7.36 (d, 2H); 7.79 (d, 2H).

### e) Preparation of

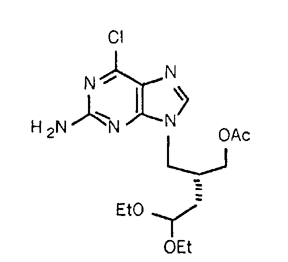

Into a 50 mL 1 neck round bottom flask was charged the product of Example 1 step d) (3.88 g, 10 mmol), anhydrous DMF (20 mL), 2-amino-4-chloro-purine (2.125 g, 12.5 mmol) and K₂CO₃ (4.83 g). The resulting suspension was stirred at 40°C under a N₂ blanket for 20 hours. The mixture was concentrated to remove most of the DMF on a rotary evaporator. The residue was diluted with EtOAc (50 mL) and H₂O (50 mL). The reaction mixture was transferred to a separatory funnel, shaken and the aqueous layer was separated. The aqueous layer was extracted with EtOAc (25 mL). The organic layers were combined and washed with 5% KH₂PO₄ (75 mL). The organic layer was separated and washed with H₂O (75 mL), brine (75 mL), dried over Na₂SO₄, filtered and concentrated in vacuo to afford 3.95 g of crude product. The crude product was slurried with 40 mL of methyl-t-butyl ether. This mixture was stirred overnight at 4°C and the mixture was filtered. The filtrate was concentrated to afford 3.35 g of the product as an oil (containing 2.6 g of the desired product based upon HPLC analysis).
300 MHz ¹H NMR (CDCI₃) δ 1.19 (m, 6H); 1.69 (2H); 1.79 (s, 1H); 2.03 (s, 3H); 2.52 (m, 1H); 3.48 (m, 2H); 3.62 (m, 2H); 4.04 (m, 2H); 4.16 (m, 2H); 4.61 (t,1H); 5.12 (bs, 2H); 7.81 (s, 1H).

### f) Preparation of

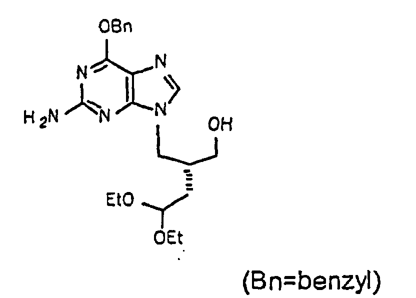

Into a 500 mL 1 neck round bottom flask was charged benzyl alcohol (136 mL), cooled to 0 °C, followed by portionwise addition of KO-t-Bu (36 g, 321 mmol). The temperature was allowed to warm to 40°C, and the mixture was stirred 20 minutes. To this mixture was added at 0°C the crude product of Example 1 step e) (24.7 g, 64.2 mmol) dissolved in 25 mL anhydrous THF and benzyl alcohol (30 mL). The temperature was allowed to slowly warm to 8°C over 2 hours. The reaction mixture was poured into 500 mL ice and was extracted with 500 mL MTBE. The organic layer was washed with 250 mL of brine, dried over Na₂SO₄, filtered and concentrated in vacuo to afford 193 g of a benzyl alcohol solution of the desired product. HPLC analysis indicated that the solution contained 25.96 g of the desired product.
300 MHz ¹H NMR (CDCI₃) δ 1.22 (m,6H); 1.55 (2H); 2.18 (m, 1H); 3.15 (m, 1H); 3.40 (m, 1H); 3.51 (m, 2H); 3.70 (m, 2H); 4.25 (m, 2H); 4.63 (t,1H); 4.90 (bs, 2H); 5.25 (m, 1H); 5.58 (s, 2H); 7.35 (m, 3H); 7.51 (m, 2H); 7.72 (s, 1H). MS = (M + H)⁺ = 416(CI).

### g) Preparation of

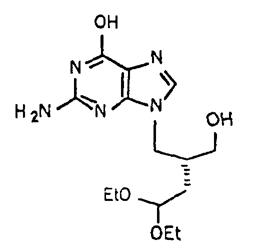

Into a 100 mL 1 neck round bottom flask was charged the crude product of Example 1 step f) (9.65 g of the benzyl alcohol solution, containing 1.30 g, 3.13 mmol of the product of Example 1, step f) dissolved in absolute EtOH (20 mL). To this was added 0.45 g of 10 % Pd/C slurried in 5 mL absolute EtOH. The reaction flask was evacuated and charged with H₂ three times with a balloon of H₂. The reaction flask was pressurized with 1 atm. H₂ and the reaction mixture was stirred overnight. The reaction mixture was filtered through a pad of diatomaceous earth to remove Pd/ C. The volatiles were removed in vacuo. The residue was mixed with 25 mL of isopropyl acetate and then concentrated in vacuo. The residue was diluted with EtOAc (10 mL), seeded with the desired product, heated to reflux and then CH₃CN (2 mL) and MTBE (35 ml) were added. The mixture was stirred for 30 minutes. The precipitate was filtered and dried to a constant weight of 600 mg of the desired product.
300 MHz ¹H NMR (d₆-DMSO) δ 1.16 (m,6H); 1.45 ( m, 1H); 1.61 ( m, 1H); 2.16 (m, 1H); 3.45 (m, 2H); 3.40 (m, 1H); 3.62 (m, 2H); 4.02 (m,2 H); 4.53 (t, 1H); 4.85 (t, 1H); 6.55 (bs, 1H); 7.75 (s, 1H). MS = (M + K)⁺ 416 (Cl).

### h) Preparation of

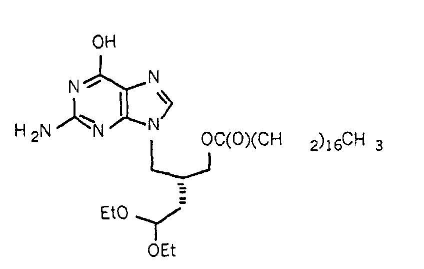

Into a 25 mL 1 neck round bottom flask was charged the product of Example 1 step g) (0.650 g, 2.0 mmol), pyridine (4 mL) and CH₂Cl₂ (2 mL), DMAP (10mg). The mixture was cooled to -5 °C and stearoyl chloride (790 mg, 2.6 mmol) dissolved in CH₂Cl₂ (0.5 mL) was added over 5 minutes. The resulting mixture was stirred 16 hours at -5°C. Absolute EtOH (0.138 g, 3.0 mmol) was added and the mixture was stirred an additional 1 hour. The reaction mixture was concentrated in vacuo. Toluene (30 mL) was added to the residue and then the mixture was concentrated in vacuo. Again, toluene (30 mL) was added to the residue and then the mixture was concentrated in vacuo. To the residue was added 1 % KH₂PO₄ (25 mL) and this mixture was extracted with CH₂CI₂ (60 mL). The organic layer was separated and was dried over Na₂SO₄, filtered and concentrated in vacuo to a constant weight of 1.65 g. The crude product was chromatographed on 40 g of SiO₂, eluting with 95/5 CH₂Cl₂ - EtOH, affording 367 mg of the desired product.
300 MHz ¹H NMR (CDCl₃) δ 0.89 (t, 3H); 1.26 (m, 30 H); 1.65 (m,3 H); 2.32 (m, 1H); 3.45 (m, 1 H); 3.60 (m, 2H); 4.08 (m, 2H); 4.60 (m, 1 H); 6.0 (bs, 2H); 7.53 (s, 1 H).

### i) Preparation of

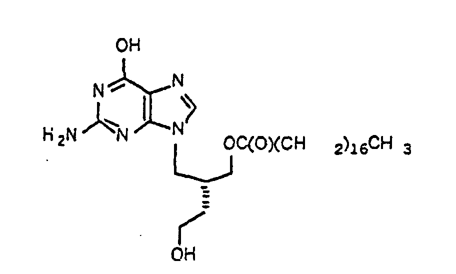

Into a 25 mL 1 neck round bottom flask was charged the product of Example 1, step h) (0.234 g, 0.394 mmol) dissolved in THF (1.7 mL). To this solution was added triflic acid (0.108 g) in H₂O(180 mg.) The mixture was stirred overnight at room temperature. To the reaction mixture was added saturated NaHCO₃ solution (10 mL), THF (5mL), CH₂Cl₂ (2 mL) and NaBH₄ (0.10 g). This mixture was stirred for 30 minutes. To the reaction mixture was added a 5% solution of KH₂PO₄ (30 mL). This mixture was extracted with 2 x 15 ml of CH₂Cl₂. The organic layers were combined and dried over Na₂SO₄, filtered and concentrated in vacuo to a constant weight of 207 mg. This material was recrystallized from EtOAc (8 mL) and CH₃CN (0.5 mL) affording 173 mg of the desired product.
300 MHz ¹H NMR (d₆-DMSO) δ 0.82(t, 3H); 1.19 (m, 30H); 1.41 (m, 4H); 2.19 (t, 2H); 2.32 (m, 1H); 3.40 (m, 2H); 3.9 (m, 4H); 4.49 (m, 1H); 6.4 (bs, 2H); 7.61 (m, 1.5H); 9.55 (m, 0.5H).

### EXAMPLE 2

### Preparation of (R)-9-[4-(N-tert-butyloxycarbonyl-L-valyloxy)-2-(stearoyloxymethyl)butyl]guanine

(R)-9-[2-(Stearoyloxymethyl)-4-(t-butyldiphenylsilyloxy)butyl]guanine (45g) and THF (950 ml) were combined in a 2L flask. Then Boc-L-valine (3.22 g, 0.25 eq) was added, followed by tetrabutylammonium fluoride (1M in THF, 89.05 mL) over 10 minutes. The clear reaction mixture was stirred at room temperature for 2 hours and 50 minutes with monitoring of the reaction progress by TLC (90/10 CH₂Cl₂/MeOH).

To the reaction mixture was added Boc-L-valine (35.43 g, 2.75 eq), DCC (36.67 g, 2.75 eq) and dimethylaminopyridine (1.1 g, 0.15 eq) in THF (25 ml). The reaction mixture was stirred at room temperature for 24 hours. DCU was filtered off and washed with CH₂CI₂. The filtrate was concentrated, and the residue was taken up in 2 litres of CH₂Cl₂ and washed with 2L of 1/2 saturated sodium bicarbonate and brine solutions. On drying and evaporation, approximately 100 g of crude product was obtained. The material was purified by silica chromatography (6000 ml of silica) using 3% MeOH/CH₂Cl₂ to 5% MeOH/CH₂CI₂ to obtain 38.22 mg of the desired product.

### EXAMPLE 3

### Preparation of (R)-9-[2-(stearoyloxymethyl)-4-(L-valyloxy)butyl]guanine

### a) (R)-9-[2-Hydroxymethyl)-4-(t-butyldiphenylsilyloxymethyl)butyl]guanine.

H2G (450.0 g, 1.78 mol) and N,N-dimethylformamide (6.4 kg) were charged into a Bucchi evaporator and the mixture warmed to dissolve the solid. The solution was concentrated to dryness under vauum at no more than 90°C. The resulting powder was transferred to a 22 litre flask with stirrer, addition funnel and and temperature probe. N,N-dimethylformamide (1.7 kg) was added followed by pyridine (3.53 kg). The resulting suspension was cooled to -10°C under nitrogen and stirred at -5°±5°C as t-butylchlorodiphenylsilane (684 g, 2.49 mol) was added dropwise. The resulting mixture was stirred at -5°±5°C until the reaction was complete (as monitored by TLC (10:1 methylene chloride/methanol) and HPLC (4.6 x 250 mm Zorbax RxC8 (5 micron); 60:40 acetonitrile-aq. NH₄OAC (0.05 M) at 1.5 ml/min; UV detection at 254 nm)). Water (16 kg) was added and the mixture was stirred for 30 minutes to precipitate the product, then the mixture was cooled to 0°C for 30 minutes. The solid was isolated by filtration and the product cake was washed with cold water and sucked dry with air to provide the crude product as an off-white solid. The crude solid was taken up in pydridine (3 kg) and concentrated under vacuum at 60°C to remove water. The dry solid residue was slurried with methanol (10 kg) at 60°C for 1-2 hours and filtered while hot. The filtrate was concentrated under vacuum and the solid residue was refluxed with isopropyl acetate (7 kg) for 30 minutes. The mixture was cooled to 20°C and filtered. The filter cake was dried under vacuum at 50°C to provide the title compound as a white solid (555 g).

### b) (R)-9-[2-(Stearoyloxymethyl)-4-(t-butyldiphenylsilyloxy)butyl]guanine.

The product of Example 3, step a) (555 g, 1.113 mol) was charged to a 50 litre Buchi evaporator. Pyridine (2.7 kg) was added dropwise to dissolve the solid and the mixture was distilled to dryness under vacuum at 60°C. The residue was taken up in fresh pyridine (2.7 kg) and transferred to a 22 litre flask with stirrer, addition funnel and temperature probe. The solution was cooled to -5°C under nitrogen. A solution of stearoyl chloride (440 g, 1.45 mol) in methylene chloride (1.5 kg) was added so as to maintain a temperature below 0°C. 4-(N,N-dimethylamino)pyridine (15 g, 0. 12 mol) was added and the mixture was stirred at -5 - 0°C for 2-4 hours until conversion was complete (as monitored by TLC (10:1 methylene chloride/methanol) and HPLC (4.6 x 250 mm Zorbax RxC8 (5 micron); 60:40 acetonitrile-aq. NH₄OAc (0.05 M) at 1.5 ml/min; UV detection at 254 nm)). At the end of the reaction, acetonitrile (8.7 kg) was added and the mixture was stirred for not less than 15 minutes to precipitate the product. The slurry was cooled to 0°C for 2 hours and the solid isolated by filtration and the filter cake washed with acetonitrile (2 kg). The desired product was obtained as a white solid (775 g).

### c) (R)-9-[4-Hydroxy-2-(stearoyloxymethyl)butyl]guanine.

A solution of the product of Example 3, step b) (765 g, 0.29 mol) in tetrahydrofuran (10 kg) was prepared in a reactor. A solution of tetra(n-butyl)ammonium fluoride in tetrahydrofuran (1.7 kg of 1 M solution, 1.7 mol) was added and the resulting clear solution was stirred at 20°± 5°C for 4 hours. Water (32 kg) was added and the resulting slurry was stirred for 1 hour and then cooled to 0°C for 30 minutes. The precipitate was isolated by filtration and the filter cake was washed successively with water (10 kg) and acetonitrile (5 kg). After drying under vacuum at 25°C, 702 g of crude product was obtained. The crude product was dissolved in refluxing THF (4.2 kg) and water (160 g), then cooled to 40°C and treated with methylene chloride (14.5 kg). The mixture was allowed to cool to 25°± 5°C for 1 hour, then it was cooled to 5°± 5°C for 1 hour to complete precipitation. The slightly off-white powder was isolated by filtration and dried under vacuum at 40°C to yield the desired product (416 g).

### d) (R)-9-[4-(N-Cbz-L-valyloxy)-2-(stearoyloxymethyl)butyl]guanine.

A solution of N-Cbz-L-valine (169 g, 0.67 mol) in dry THF (750 ml) was prepared in a 2 litre flask with mechanical stirrer, thermometer and addition funnel. A solution of dicyclohexylcarbodiimide (69.3 g, 0.34 mol) in THF (250 ml) was added over 5 minutes and the resulting slurry was stirred at 20°± 5°C for 2 hours. The slurry was filtered and the filter cake was washed with THF (300 ml). The filtrate and wash were charged to a 3 litre flask with stirrer and thermometer. The product of Example 3, step c) (116 g, 0.22 mol) was added as a solid, with a rinse of THF (250 ml). 4-(N,N-dimethylamino)pyridine (2.73 g, 0.022 mol) was added and the white slurry stirred at 20°± 5°C. Within 15 minutes, the solids were all dissolved and the reaction was complete within 1 hour (as determined by HPLC: 4.6 x 250 mm Zorbax RxC8 column; 85:15 acetonitrile- 0.2% aq. HCICO₄ at 1 ml/min.; UV detection at 254 nm; starting material elutes at 4.1 min. and product elutes at 5.9 min.). The reaction was quenched by addition of water (5 ml) and the solution was concentrated under vacuum to leave a light yellow semisolid. This was taken up in methanol (1.5 litres) and warmed to reflux for 30 minutes. The solution was cooled to 25°C and the precipitate was removed by filtration. The filtrate was concentrated under vacuum to leave a viscous, pale yellow oil. Acetonitrile, (1 L) was added and the resulting white suspension was stirred at
20°± 5°C for 90 minutes. The crude solid product was isolated by filtration, washed with acetonitrile (2 x 100 ml) and air-dried overnight to provide the desired product as a waxy, sticky solid (122 g). This was further purified by crystallization from ethyl acetate (500 ml) and drying under vacuum at 30°C to provide the desired product as a white, waxy solid (104 g).

### e) (R)-9-[4-(L-valyloxy)-2-(stearoyloxymethyl)butyl]guanine.

A solution of the product of Example 3, step d), (77 g) in warm (40°C) ethanol (2.3 L) was charged to an hydrogenation reactor with 5% Pd-C (15.4 g). The mixture was agitated at 40°C under 40 psi hydrogen for 4 hours, evacuated and hydrogenated for an additional 4-10 hours. The catalyst was removed by filtration and the filtrate was concentrated under vacuum to provide a white solid. This was stirred with ethanol (385 ml) at 25°C for 1 hour, then cooled to 0°C and filtered. The filter cake was dried with air, then under vacuum at 35°C to yield the title compound as a white powder (46 g).

### EXAMPLE 4

### 9-R-(4-Hydroxy-2-(L-valyloxymethyl)butyl)guanine

a) To a solution of 9-R-(4-(tert-butyldiphenylsilyloxy)-2-(hydroxymethyl)butyl)guanine (695 mg, 1.5 mmole) in DMF (30 ml) were added N-Boc-L-Valine (488 mg, 2.25 mmole), 4-dimethylamino pyridine (30 mg, 0.25 mmole) and DCC (556 mg, 2.7 mmole). After 16 hr, the reaction was recharged with N-Boc-L-valine (244 mg) and DCC (278 mg), and was kept for an additional 5 hours. The reaction mixture was filtered through Celite and poured into sodium hydrogen carbonate aqueous solution, and then it was extracted with dichloromethane. The organic phase was evaporated and purified by silica gel column chromatography, giving 950 mg of the N-protected monoamino acyl intermediate.
b) The above intermediate (520 mg, 0.78 mmole) was dissolved in THF (15 ml). To the solution was added hydrogen fluoride in pyridine (70%/30%, 0.34 ml). After two days, the solution was evaporated and coevaporated with toluene. Purification by silica gel column chromatography gave 311 mg of the protected monoamino acyl compound.
   ¹H-NMR (DMSO-d₆): δ 10.41(s, 1H), 7.59 (1H), 6.26 (br s, 2H), 4.32 (t, 1H), 3.95 (m, 5H), 3.46 (m, 2H), 2.41 (m, 1H), 2.06 (m, 1H), 1.45 (m, 2H), 1.39 (s, 9H), 0.90 (d, 6H).
c) The product of step b) (95 mg, 0.21 mmole) was treated with a mixture of trifluoroacetic acid (4 ml) and dichloromethane (6 ml) for 1 hr. The solution was evaporated and freeze-dried, to give 125 mg of the unprotected monoaminoacyl product.
   ¹H-NMR (D₂O): δ 8.88 (s, 1H), 4.32 (m, 4H), 3.96 (d, 1H), 3.68 (m, 2H), 2.63 (m, 1H), 2.22 (m, 1H), 1,73 (m, 2H), 1.00 (m, 6H).

### EXAMPLE 5

### Alternative preparation of (R)-9-[(2-stearoyloxymethyl)-4-(L-valyloxy)butyl]guanine

### a) Preparation of (R)-2-Amino-6-chloro-9-[4,4-diethoxy-2-(hydroxymethyl)butyl] purine

The product of Example 1, step e) (200 g) was dissolved in methanol (670 mL) and 20% aqueous K₂CO₃ (43 g K₂CO₃ in 166 mL H₂O) was added. The mixture was stirred at 25±5°C for 30 minutes. The reaction mixture was then cooled to 0-5°C for about 20 minutes, when a precipitate formed. Water (500mL) was added and the slurry was mixed at 5± 5°C for 15 minutes. The resulting solid was isolated by filtration and the filter cake was washed with water (100 mL) and dried under vacuum at 20°C to provide the desired product as a pale yellow powder (81 g). m.p. 156-158°C.
300 MHz ¹H NMR (DMSO-d₆) δ 1.04 (m, 6H); 1.36 (m, 1H); 1.55 (m, 1H); 2.10 (m, 1H); 3.40 (m, 6H); 4.06 (m, 2H); 4.48 (t, 1H); 4.78 (t, 1H); 6.93, (br s, 2H); 8.10 (s, 1H).

### b) Preparation of (R)-9-[4,4-diethoxy-2-(hydroxymethyl)butyl]guanine.

To the product of Example 5, step a) (22.5 kg, 65.4 moles) was added an aqueous solution of KOH (prepared by dissolving 12.9 kg of KOH in 225 kg of water). This mixture was refluxed for 16 hours. The reaction was cooled to about room temperature and filtered into a larger reactor equipped with a pH electrode standardized to pH 7-10. The filtered solution was cooled to 5°C and the product precipitated by slow addition of dilute acetic acid solution (prepared by mixing glacial acetic acid (12.6 kg, 210 moles) with 75 kg of water and cooling the mixture to 5°C) until the pH is between 7.5 and 9.0 (target 8.5). The resulting slurry was immediately filtered and the filter cake was recharged back to the reactor. The reactor was charged with 225 kg of distilled water. The mixture was heated to not more than 50°C for 30 minutes, then cooled to 15±10°C and stirred for 30 minutes. The resulting precipitate was filtered by vacuum filtration, rinsed with 50 kg of distilled water and dried in a vacuum oven at not more than 45°C for not less than 8 hours to provide the desired product as a tan solid.

### c) Preparation of Stearoyl-pivaloyl mixed anhydride.

To 22.4 kg of stearic acid (78.7 moles) in 156.4 kg of toluene was added 8.2 kg of triethylamine (81.0 moles). The internal temperature of the resulting slurry was lowered to -5°C, then 9.52 kg of pivaloyl chloride (79.0 moles) was slowly added maintaining an internal temperature of not more than 5°C. The slurry was stirred for 2 hours at 5°C, then warmed to 20°C and stirred for 4 hours. The triethylammonium hydrochloride precipitate was filtered and washed with 36.6 kg, 35.5 kg and 37.9 kg of toluene. The filtrate was concentrated at not more than 60°C internal temperature and 61.1 kg of heptane was added, followed by cooling the slurry to -15 to -10°C. After 4 hours of stirring, the resulting solid was collected by vacuum filtration, blown dry for 1 hour with nitrogen and dried in a vacuum oven at room temperature for 1.5 hours to provide the desired product as white crystals (18.9 kg). A further 2.7 kg of the desired product was obtained by concentrating the mother liquors under vacuum and adding 41.1 kg of heptane. The resulting slurry was cooled to -15 to -10°C for 4 hours, filtered, blown dry with nitrogen for 1 hour and the product dried in a vacuum oven at room temperature.

### d) Preparation of (R)-9-[4,4-diethoxy-2-(stearoyloxymethyl)butyl]guanine.

The product of Example 5, step b) (3.9 kg, 11.9 moles), the product of Example 5, step c) (5.2 kg, 13.6 moles) and 300 g of 4-dimethylaminopyridine (2.4 moles) were combined in 103.3 kg of THF at room temperature. After mixing for 16 hours, water (3 kg) was added. After mixing for 45 minutes, the solution was distilled at not more than 45°C internal temperature. Ethyl acetate (62.9 kg) was charged and the solution was redistilled at not more than 45°C internal temperature. Acetone (56 kg) was then added and the slurry heated to reflux (56°C) for 15 minutes. The resulting clear solution was cooled to room temperature (not more than 15°C/hour). After 4 hours at room temperature, the resulting precipitate was filtered and rinsed with acetone (17 kg).

The mother liquors were concentrated under vacuum at not more than 45°C. Ethyl acetate (260 kg) and water (72.1 kg) were charged. The biphasic mixture was stirred and then allowed to settle. The organic phase was separated and was distilled. Ethyl acetate (200 kg) was added and the solution was redistilled. Acetone (101 kg) was charged, the solution heated to reflux (56°C) for 15 minutes and then the solution was cooled to room temperature (not more than 15°C/hour) and the precipitate was filtered. The product was washed with acetone (19 kg, 15 kg and 15 kg), blown dry with nitrogen for 1 hour and then dried under vacuum at not more than 40°C for approximately 6 hours to yield the desired product (3.1 kg).

### e) Preparation of (R)-9-[4-hydroxy-2-(stearoyloxymethyl)butyl]guanine.

The product of Example 5, step d) (3.0 kg) was slurried in THF (46 L) at 20°C. A solution of trifluoromethanesulfonic acid (2.25 kg) in 2.25 kg of water (prepared by slowly adding the acid to cold water) was added and the reaction mixture was stirred at 22°C for 2 hours. The reaction mixture was cooled to 15°C and quenched with a solution of NaHCO₃ (1.5 kg) in water (5.3 kg). Borane t-butylamine complex (powder, 340 g) was added in four portions and then the reaction temperature was increased to 35°C and stirred for 12 hours. The reaction mixture was added to a solution of 320 g of concentrated HCI (37% aq.) in 115 kg of tap water at 5°C. This mixture was stirred for 30 minutes and the resulting precipitate was filtered and washed with acetonitrile (15 kg). The solids were reprecipitated once or twice from acetone (35 kg). A final precipitation was accomplished by dissolving the product in THF (24 kg) at 65°C, adding water (1.3 kg), cooling to 30°C and then adding methylene chloride (105 kg). The resulting slurry was cooled to 10°C and the precipitate was filtered to provide the desired product.

### f) Preparation of (R)-9-[4-(N-benzyloxycarbonyl-L-valyloxy)-2-(stearoyloxymethyl)butyl]guanine.

A solution of dicyclohexylcarbodiimide (1500 g, 7.27 moles) in THF (7 L) was added to a reactor containing a mixture of N-carbobenzyloxy-L-valine (3630 g, 14.5 moles) in THF (20 L). The resulting mixture was stirred at 20 ±5°C for 1-2 hours. The product of Example 5, step e) (2500 g, 4.81 moles) and 4-dimethylaminopyridine (59 g, 0.48 moles) were charged to a second reactor. To this second reactor was filtered the THF mixture from the first reactor, followed with a rinse of THF (15 L). The resulting mixture was stirred at 20 ±5°C for 1-3 hours. Water (600 mL) was added and the solution was concentrated under vacuum at not more than 45°C. The residual oil was taken up in ethyl acetate (14 L) and filtered. The filtrate was washed successively with 10% aqueous sodium bicarbonate (2 x 14 L) and 10% brine (14 L). The organic phase was concentrated under vacuum and the residue was dissolved in methanol (10 kg) at 50-60°C. The warm solution was added gradually to a mixture of acetonitrile (30 kg) and water (13 kg) at ambient temperature. The mixture was stirred 1 hour at 15°C, then filtered to isolate the crude product, which was dried at 40°C under vacuum to provide the desired product as a white solid (3.9 kg).

### g) Preparation of (R)-9-[(2-stearoyloxymethyl)-4-(L-valyloxy)butyl]guanine.

A hydrogenation reactor was charged with 10% Pd-C (400 g) and the product of Example 5, step f) (2.4 kg). Absolute ethanol (52 L) was added and the mixture was warmed to 40°C and hydrogenated at 30-40 psi for 3-5 hours. On completion of the reaction, the catalyst was removed by filtration through diatomaceous earth and the filter cake was rinsed well with ethanol (30 L). The combined filtrates were concentrated under vacuum at not more than 60°C to leave a white solid residue. This was dissolved in isopropanol (15 L) and isopropyl acetate (60 L) at reflux and then allowed to cool to room temperature over 4 hours. After cooling for 3 hours at 15 ±10°C, the precipitate was isolated by filtration, washed with isopropyl acetate (6 L) and dried under vacuum at 40°C to provide the desired product as a white powder (864 g).

### EXAMPLE 6

### Alternative preparation of (R)-9-[(2-stearoyloxymethyl)-4-(L-valyloxy)butyl]guanine

### a) Preparation of (2R)-4,4-Diethoxy-2-stearoyloxymethyl-butanol.

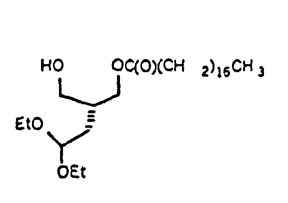

Vinyl stearate (17.76 g. 0.057 moles) was charged to a 100 mL round bottom flask with a magnetic stir bar. The flask was immersed with stirring in a 35 °C oil bath. The product of Example 1, step b) (10.0 g, 0.052 moles) and Lipase Amano PS-30 (0.20g) were added and stirred for four hours at 35°C. The reaction was diluted with hexane (260 mL) and MTBE (115 mL) and filtered through celite. The filtrate was washed twice with water (100 mL), dried with Na₂SO₄, and concentrated to provide the desired product (26.21 g) as a clear oil that forms a wet solid on standing at room temperature.

### b) Preparation of (2S)-4,4-Diethoxy-2-stearoyloxymethyl-butyl toluenesulfonate.

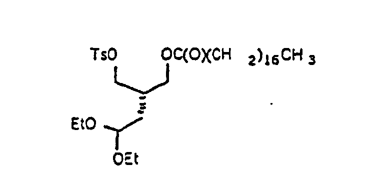

The product of Example 6, step a) (26.21 g, 0.057 mol) was dissolved in methylene chloride (75 mL) and charged into a 250 mL 3 necked flask equipped with a magnetic stir bar, condenser, N₂ inlet, and temperature probe. Triethylamine (14.4 g) was added followed by p-toluenesulfonyl chloride (16.3 g). The flask was purged with N₂ and heated to reflux ( 46°C). The reaction was stirred at reflux 6 hours. The reaction was cooled to room temperature. Water (10 mL) was added and the reaction was stirred vigorously for 16 hours. The reaction mixture was poured into a 1 L separatory funnel containing ethyl acetate (350 mL) and water (350 mL). The organic layer was separated and washed with 7% (w/w) aq. sodium bicarbonate (100 mL. The organic layer was then washed with 23% (w/w) aq. sodium chloride (100 mL). The organic layer was dried with Na₂SO₄ and filtered. The solution was concentrated to give the desired product (29.4 g) as an oil that formed a wet solid when cooled to room temperature.

### c) Preparation of (3S)-3-stearoyloxymethyl-4-toluenesulfonyloxy-butyraldehyde.

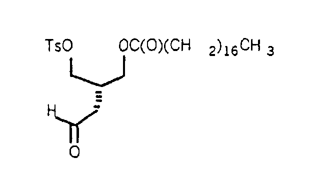

The product of Example 6, step b) (29.38 g, assayed at 23.12 g, 0.037 moles) was dissolved in THF (90 mL) and charged into a 250 mL round bottomed flask equipped with a magnetic stir bar and a temperature probe. Charged water (38 mL) and cooled to 10 °C. Trifluoroacetic acid (55 mL) was poured in and the mixture was stirred for 25 minutes. The reaction mixture was poured into a 2 L separatory funnel containing 20% (w/w) K₂CO₃ solution (690 g), ice (600 g), and ethyl acetate (500 mL). The upper organic layer was separated. The aqueous layer was extracted a second time with ethyl acetate (500 mL). The combined organic extracts were washed with 23% (w/w) NaCI solution. The organic layer was separated, dried with Na₂SO₄ and filtered. The solution was concentrated to 21.5 g of an oil, dissolved in heptane (150 mL), and stirred slowly (crystals formed after 10 minutes). The slurry was stirred 15 hrs. at ambient temperature, filtered and washed with ambient heptane (20 mL). The desired product was obtained as white crystals which were dried to a constant weight of 12.3 g.

### d) Preparation of (2S)-4-N-Carbonylbenzyloxy-L-valinyloxy-2-stearoyloxymethyl-butyl toluenesulfonate.

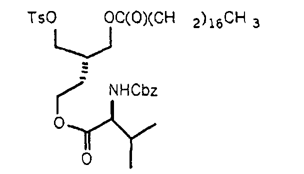

The product of Example 6, step c) (11.91 g, 0.022 mol) was charged to a 250 mL shaker bottle. THF (120 mL) and RaNi (17.8 g) were added. The reaction was pressurized to 4 atm. with H₂. The reaction was shaken for 1.5 hours. The reaction was filtered and washed with 20 mL THF. The filtrate is diluted with 100 mL of CH₂Cl₂, dried with Na₂SO₄, filtered, and washed with 25 ml CH₂Cl₂. The filtrate was charged to a 500 mL 3 necked flask equipped with a magnetic stir bar and N₂ inlet. N-Cbz-L-valine (13.88 g, 0.055 moles), 1,3-dicyclohexylcarbodiimide (11.37 g, 0.055 moles), and 4-dimethylaminopyridine (0.40 g, 0.003 moles) were added and the reaction was stirred for 1 hr. The reaction mixture became heterogeneous after several minutes. The reaction was filtered and washed with CH₂CI₂ (50 mL). The filtrate was diluted with ethyl acetate (600 mL) and washed twice with 7% (w/w) NaHCO₃ solution (100 mL). The organic layer was then washed twice with 5% (w/w) KH₂PO₄ solution (100 mL). The organic layer was washed with 7% (w/w) NaHCO₃ solution (100 mL), then dried with MgSO₄ and filtered. The solution was concentrated to 19.46 g of oily solids. The solid was dissolved in 30 mL of 8:2 hexanes:ethyl acetate and chromatographed in two parts. Each half was chromatographed on a Flash 40M silica gel cartridge (90 g of 32-63 µm, 60Å silica 4.0 cm X 15.0 cm) and eluted with 8:2 hexanes:ethyl acetate at 25 ml/min. 25 ml fractions were collected. Fractions were analyzed by TLC. Fractions 10-22 contained pure product in the first run and fractions 9-26 contained pure product in the second run. The fractions were combined and concentrated to provide the desired product as a clear viscous oil (12.58 g).

### e) Preparation of 6-Benzyloxy-2-amino-purine.

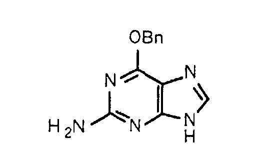

60% Sodium hydride in mineral oil (2.36 g, 0.059 moles) was charged to a 500 mL 3-neck flask equipped with magnetic stirring, temperature probe, condenser, and N₂ inlet. Toluene (250 mL) was added. Benzyl alcohol (50 mL) was added dropwise over 30 minutes. After addition of benzyl alcohol, the reaction was stirred 10 minutes. Then 6-chloro-2-aminopurine (5.00 g, 0.029 moles) was added and the reaction mixture was heated to reflux (115°C) for 4.5 hours. The reaction mixture was filtered hot through a coarse glass fritted funnel and 11.65 g of wet off-white solids were obtained. The wet solids were triturated with CH₂CI₂ (100 mL) and water (100 mL). After 10 minutes of stirring the solids had dissolved. The aqueous layer was separated and the pH was lowered to 9 over 3 minutes with 6 M HCI. A white solid precipitate formed. The slurry was filtered, washed with water (50 mL), and dried (*in vacuo* at 50° C) to a constant weight to provide the desired product as off-white crystals (5.15 g).

### f) Preparation of (R)-9-[(2-stearoyloxymethyl)-4-(N-benzyloxycarbonyl-L-valyloxy)butyl]guanine.

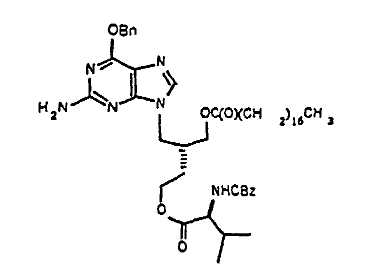

The product of Example 6, step e) (2.40 g, 0.0099 moles) was charged to a 100 mL round bottom flask equipped with magnetic stirring and a N₂ inlet. DMF (6 mL) and potassium carbonate (6.27 g) were added. The mixture was stirred at room temperature for 30 minutes. The product of Example 6, step d) (7.02 g, 0.0091 moles) was dissolved in DMF (21 mL) and added to the mixture. The flask was immersed in a 70°C oil bath and stirred 24 hours. The reaction was cooled to ambient temperature and poured into a 500 mL separatory funnel containing ethyl acetate (135 mL) and 5% (w/w) KH₂SO₄ solution (135 mL). The top organic layer was kept and washed with 7% (w:w) NaHCO₃ solution (100 mL). The organic layer was dried with MgSO₄ and filtered. The solution was concentrated to 9.79 g of oily solids. This was triturated in 50 mL of 1:1 hexanes:ethyl acetate, filtered, and concentrated to 9.10 g of yellow oil. The oil was dissolved in 20 mL of 1/1 hexanes-ethyl acetate and chromatographed on a Flash 40M silica gel cartridge (90 g of 32-63 µm, 60Å silica, 4.0 cm X 15.0 cm) eluted with 6:4 hexanes:ethyl acetate at 25 ml/min. 25 ml fractions were collected. Fractions were analyzed by TLC. Fractions 27-92 contained pure product by TLC. The pure fractions were combined and concentrated to yield the desired product as an oil (2.95 g).

### g) Preparation of (R)-9-[(2-stearoyloxymethyl)-4-(L-valyloxy]butyl]guanine.

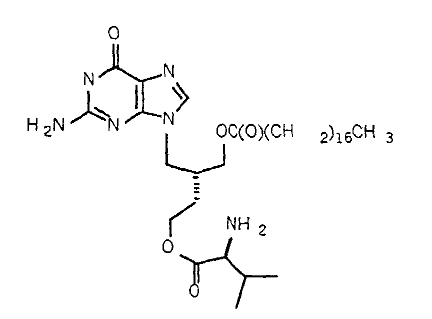

The product of Example 6, step f) (2.63 g, 0.0031 moles) was dissolved in ethanol (50 mL) and charged into a 500 mL round bottom flask. 10% Pd/C (0.5 g) was slurried in ethanol (20 mL) and added to the flask. The reaction was stirred under H₂ (1 atm from balloon) for 1.5 hours. The slurry was heated briefly to dissolve any solids, filtered through celite, and washed with hot ethanol (50 mL). The filtrate was concentrated to give 1.752 g of white solid. The solid was dissolved in isopropyl alcohol (10 mL) and isopropyl acetate (42 mL) at 70°C. The solution was cooled to 15 °C over 2 hours and stirred at 15 °C for 12 hours. The solution was cooled to 0 °C over 30 minutes and stirred for 1 hour. The slurry was filtered and washed with isopropyl acetate (10 mL). The solid was dried *in vacuo* at 50 °C to provide the desired product (0.882 g).

The mother liquors were concentrated to give 0.55 g of white solid which was dissolved in isopropyl alcohol (3mL) and isopropyl acetate (16 mL) at 75°C. The solution was cooled to 15 °C for 2 hours, then filtered and dried as above to to provide an additional 0.181 g of the desired product.

### EXAMPLE 7

### Alternative preparation of Ethyl 4,4-Diethoxy-2-ethoxycarbonyl butyrate

This example shows a process for purifying a compound of the formula 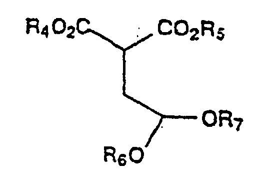 wherein R₄ and R₅ are loweralkyl or benzyl and R₆ and R₇ are loweralkyl or benzyl or R₆ and R₇ taken together are -CH₂CH₂-, -CH₂CH₂CH₂-, or -CH₂CH₂CH₂CH₂-, the process comprising the reaction of the above depicted compound with a dilute aqueous base.

To a suspension of sodium ethoxide (20 g, 0.294 moles) in dimethylformamide (68 g) was added diethyl malonate (49 g, 0.306 moles) during 13 minutes. After the addition was complete, the mixture was heated to 110°C and bromoacetaldehyde diethyl acetal (40 g, 0.203 moles) was added over 1 hour and 45 minutes. After the addition was complete, the mixture was heated at 110°C for 7 hours. The reaction mixture was cooled to room temperature and methyl t-butyl ether (160 g) and water (100g) were added and the mixture was stirred for 15 minutes. The organic layer was separated and treated with 7% aqueous potassium hydroxide solution (155 g). The layers were separated and the organic layer was washed with water (100 g) and then with brine (60 g). The organic layer was concentrated to give the crude desired product. The crude product was heated under house vacuum (approximately 45 mm of Hg) at 160-170°C (bath temperature) to distill off the volatile impurities, providing 43.6 g of the desired product.

### EXAMPLE 8

### Alternative preparation of (R)-9-[4,4-diethoxy-2-(hydroxymethyl)butyl]guanine.

To a 100 mL one neck flask was added the product of Example 5 a) (5 g, 0.0145 moles), followed by the addition of a solution of KOH (2.05 g, 0.0445 moles) in water (20 mL). The mixture was stirred at reflux for 16-20 hours. Then the reaction mixture (at reflux) was adjusted to pH 7.0 by the addition of acetic acid. The reaction mixture was then cooled to room temperature and stirred for 30 minutes. The resulting precipitate was collected by filtration and washed with water (5 mL). The resulting solid was dried overnight at not more than 50°C to provide 4.45 g of the desired product.

### EXAMPLE 9

### Alternative purification of (R)-9-[4-hydroxy)-2-(stearoyloxymethyl)butyl]guanine as the (S)-(+)-camphorsulfonic acid salt

This example shows a process for purifying a compound of the formula 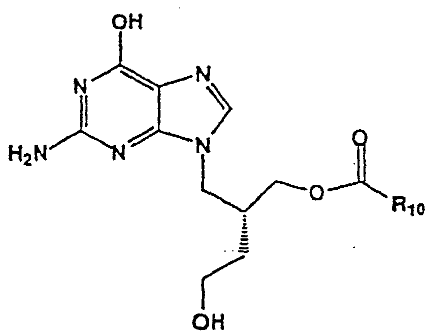 wherein R₁₀ is C₃-C₂₁ saturated or monounsaturated, optionally substituted alkyl, the process comprising the reaction of the above depicted compound with a chiral organic sulfonic acid.

In a 250 mL round bottom flask was placed the product of Example 1 i) (13.0 g) and (1S)-(+)-10-camphorsulfonic acid (5.85 g). Heptane (50 mL) was added and the mixture was stirred for 15 minutes. Then tetrahyrofuran (THF; 50 mL) was added and the mixture was stirred for 5 hours. The resulting precipitate was collected by filtration and washed with heptane (100 mL). The resulting solid was dried under vacuum at 45°C to provide the desired product (11.3 g). HPLC analysis of the product indicated 98.76% e.e.

### EXAMPLE 10

Preparation of

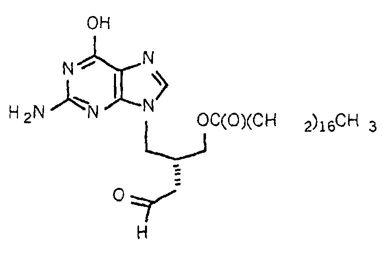

A 50 mL round bottom flask was charged with the product of Example 1 h) (1.0 g, 1.7 mmol), THF (20 mL), H₂O (1 mL), and Amberlyst 15 resin (1.0 g). The solution was then heated to 65 °C for 3 hours. The solution was then filtered hot and the resin was washed with THF (2 x 10 mL). The solvent was then removed under vacuum to give the desired product (0.74 g, 84%).

### EXAMPLE 11

### Alternative preparation of (R)-9-(4-hydroxy)-2-(stearoyloxymethyl)butyl]guanine

A 100 mL round bottom flask was charged with the product of Example 1 h) (2.45 g, 4.14 mmol), THF (25 mL), H₂O (1 mL) and Amberlyst 15 resin (2.5 g). The solution was then heated to 65 °C for 3 hours. The solution was then filtered hot and the resin was washed with THF (2 x 15 mL). The solution of the crude aldehyde was cooled to room temperature and a solution of borane t-butylamine complex (0.3 g, 3.45 mmol), in THF/H₂O (1/1 20 mL) was added dropwise to the aldehyde solution. The solution was stirred at room temperature for 1.5 hours, and the reaction was then quenched by addition of H₂O (100 mL). After stirring at room temperature for an additional 30 min., the precipitate was isolated by filtration and dried to give 1.00 g (47%) of the desired product.

### EXAMPLE 12

### Alternative preparation of (R)-9-[4-(N-benzyloxycarbonyl-L-valyloxy)-2-(stearoyloxymethyl)butyl]guanine

### a) N-Carbobenzyloxy-L-valine Anhydride

A solution of dicyclohexylcarbodiimide (5 kg, 24 moles) in acetonitrile (17.5 kg) was added to a reactor containing a solution of N-carbobenzyloxy-L-valine (12.5 kg, 50 moles) in acetonitrile (200 kg). The mixture was stirred at 5 +/- 5 °C for 6 hours and the resulting solid was filtered off.

The filtrate was concentrated under vacuum at not more then 45°C and the residue was dissolved in toluene (50 kg) at 40°C. Heptane (50 kg ) was added and the mixture was cooled to 15+/- 5°C. The precipitate was filtered off and dried to give 10.2 kg of the desired product.

### b) (R)-9-[4-(N-benzyloxycarbonyl-L-valyloxy)-2-(stearoyloxymethyl)butyl]-guanine

A mixture of (R)-(-[4-hydroxy-2-(stearoyloxymethyl)butyl]guanine (5.2 kg, 10 moles), N-CBZ-L-valine anhydride (6.3 kg, 13 moles), 4-dimethylaminopyridine (60g, 0.5 moles) and tetrahydrofuran (67 kg) was stirred for 2-4 hours at 25 +/- 5°C. Water (2 kg) was added and the mixture was concentrated under vacuum at not more then 45°C. The residue was dissolved in ethyl acetate (58 kg) and extracted with 10% aqueous sodium bicarbonate (2 x 50 kg) and water (1 x 50 kg). The ethyl acetate solution was concentrated under vacuum and the residue was dissolved in methanol (20 kg) at 50+/-5°C. The solution was cooled to 20+/-5°C and diluted with acetonitrile (50 kg) and water (3 kg). The precipitate was filtered off and dried under vacuum to give the desired product (5.3 kg).

### EXAMPLE 13

### Alternative preparation of (R)-9-[4,4-diethoxy-2-(stearoyloxymethyl)butyl]-guanine

To a stirred solution of stearic acid (1.05 g) and N-methylmorpholine (0.62 g) in THF (13 mL) at 0-4°C was added a solution of p-tosyl chloride (0.67 g) in THF (2 mL) at -3 to -4°C. The mixture was stirred at room temperature for 3 hours. The product of Example 1g) (1.0 g) and 4-dimethylaminopyridine (75 mg) were added and the slurry was stirred at room temperature for 5 days and quenched with 135 mL of water. The mixture was stirred overnight and the precipitate was filtered and washed with water. The wet filter cake was dried under vacuum (40°C) to give the desired product (1.3 g) as a light yellow powder.

### EXAMPLE 14

### Alternative preparation of (R)-9-(4,4-diethoxy-2-(hydroxymethyl)butyl]guanine.

The product of Example 5 a) (10.0 g, 29.1 mmoles) was added to a solution of sodium hydroxide (2.33 g, 5.82 mmoles) in water (200 mL). A solution of trimethylamine (6.61 mL of 40 wt. % solution in water, 43.6 mmoles) was charged to the suspension. The heterogeneous mixture was stirred at room temperature overnight. The reaction was diluted with water (50 mL) and then extracted with ethyl acetate (200 mL). The water layer was charged with a saturated solution of ammonium sulfate (300 mL). The mixture was stirred at room temperature for 30 hours and the resulting precipitate was filtered. The filter cake was washed with ethyl acetate (100 mL). The product was dried in a vacuum oven (high house vacuum, 45°C) overnight to provide the desired product (7.88 g).

## Claims

1. A compound of the formula: 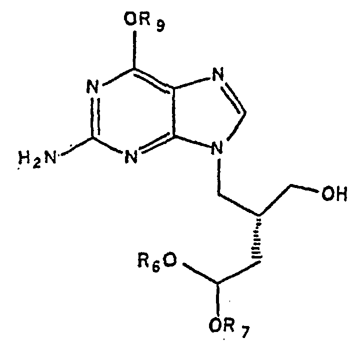 wherein R₆ and R₇ are C₁-C₇ alkyl or benzyl or R₆ and R₇ taken together are -CH₂CH₂-, -CH₂CH₂CH₂- or -CH₂CH₂CH₂CH₂- and R₉ is -H or an alcohol protecting group selected from substituted methyl ethers, tetrahydropyranyl ethers, substituted ethyl ethers, silyl ethers, and carboxylic acid esters, wherein if R₉ is -H the corresponding keto-tautomeric form is included.

2. The compound of claim 1, wherein the substituted methyl ether is methoxymethyl, benzyloxymethyl, 2-methoxyethoxymethyl, 2-(trimethylsilyl)ethoxymethyl, t-butyl, benzyl, or triphenylmethyl; wherein the substituted ethyl ether is 2,2,2-trichloroethyl; wherein the silyl ether is trimethylsilyl, t-butyldimethylsilyl, or t-butyldiphenylsilyl; and wherein the carboxylic acid ester is acetate, propionate, or benzoate.

3. The compound of claim 1 or 2, wherein R₆ and R₇ are -CH₃ or -CH₂CH₃ or R₆ and R₇ taken together are -CH₂CH₂-, -CH₂CH₂CH₂- or -CH₂CH₂CH₂CH₂- and R₉ is benzyl.

4. The compound of claim 3, wherein R₆ and R₇ are -CH₂CH₃ and R₉ is benzyl.

5. The compound of claim 1, wherein R₆ and R₇ are -CH₂CH₃ and R₉ is -H, the corresponding keto-tautomeric form being included.

6. A process for the preparation of the compound of any of the preceding claims, the process comprising the step of reacting a compound of the formula: 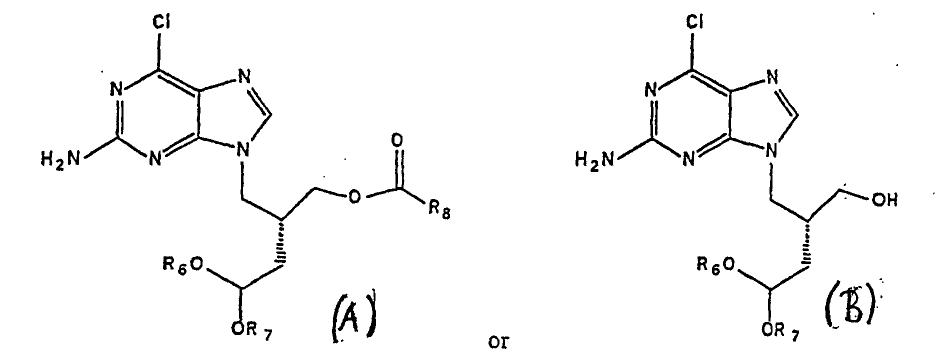 wherein R₆ and R₇ are as defined above and R₈ is C₁-C₂₁ saturated or monounsaturated, optionally substituted alkyl, with an inorganic base or, in the case where R₉ is an alcohol protecting group, with an alcohol R₉OH in the presence of a base which is potassium t-butoxide, potassium carbonate, NaH, KH, or lithium diisopropylamide, optionally followed by deprotection of R₉ to the corresponding alcohol.

7. The process of claim 6, wherein in the compound of formula (A) R₆ and R₇ are -CH₂CH₃ and R₈ is methyl, the process comprising the step of reacting the compound of formula (A) with potassium carbonate.

8. The process of claim 6, wherein the inorganic base is KOH or NaOH.

9. A compound of the formula 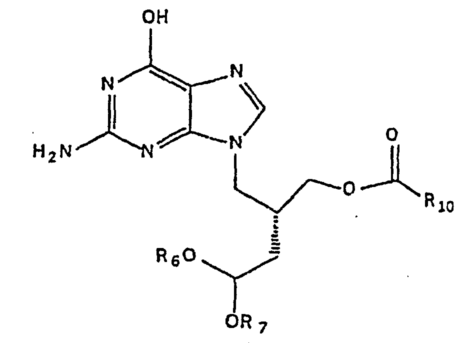 wherein R₆ and R₇ are C₁-C₇ alkyl or benzyl or R₆ and R₇ taken together are -CH₂CH₂-, CH₂CH₂CH₂- or -CH₂CH₂CH₂CH₂-, and R₁₀ is C₃-C₂₁ saturated or monounsaturated, optionally substituted alkyl, wherein the up to five similar or different substituents are independently selected from -OH, C₁-C₆ alkyl, C₁-C₆ alkoxy, C₁-C₆ alkoxy C₁-C₆ alkyl, C₁-C₆ alkanoyl, amino, halo, cyano, azido, oxo, mercapto, and nitro; and the ketomeric tautomers of these compounds.

10. The compound of claim 9, wherein R₆ and R₇ are -CH₃ or - CH₂CH₃ or R₆ and R₇ taken together are -CH₂CH₂-, -CH₂CH₂CH₂- or -CH₂CH₂CH₂CH₂-, and R₁₀ is -(CH₂)₁₆CH₃.

11. The compound of claim 10, wherein R₆ and R₇ are -CH₂CH₃ and/or R₁₀ is -(CH₂)₁₆CH₃.

12. A process for the preparation of the compound of any of claims 9 to 11, the process comprising the step of reacting a compound of the formula: 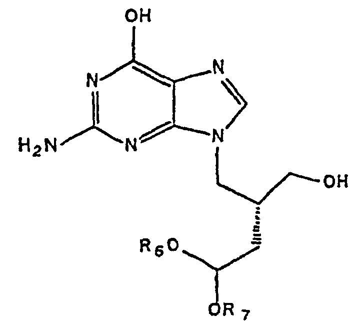 or its ketomeric tautomer, wherein R₆ and R₇ are as defined above, with R₁₀ COOH or an activated derivative thereof.

13. The process of claim 12, wherein the activated derivative of R₁₀ COOH is R₁₀C(O)OS(O)₂R₃₀, wherein R₃₀ is C₁-C₇ alkyl, phenyl or toluyl, or R₁₀C(O)OC(O)R₁₀ or R₁₀C(O)OC(O)R₁₀ₐ, wherein R₁₀ₐ is C₁-C₇ alkyl, and wherein R₁₀ is -(CH₂)₁₆CH₃.

14. A process for the preparation of a compound of the formula: 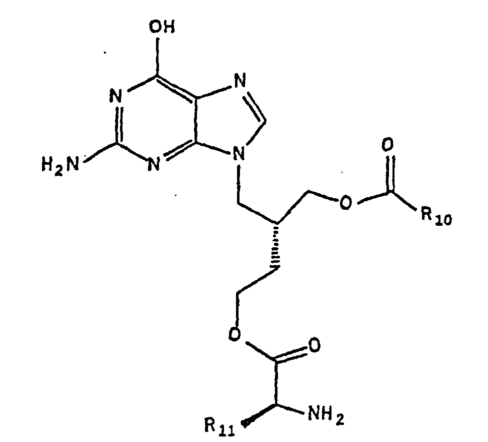 wherein R₁₀ is C₃-C₂₁ saturated or monounsaturated, optionally substituted alkyl and R₁₁ is isopropyl or isobutyl, the process comprising one of the three following sequences of steps:
1a) deprotecting the acetal of the compound of the formula: 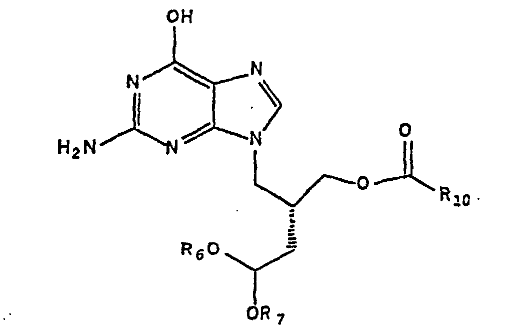 wherein R₆ and R₇ are C₁-C₇ alkyl or benzyl or R₆ and R₇ taken together are -CH₂CH₂-, -CH₂CH₂CH₂- or -CH₂CH₂ CH₂CH₂-; and
1b) reducing the aldehyde substituent of the product of step 1a) to provide the alcohol of the formula: 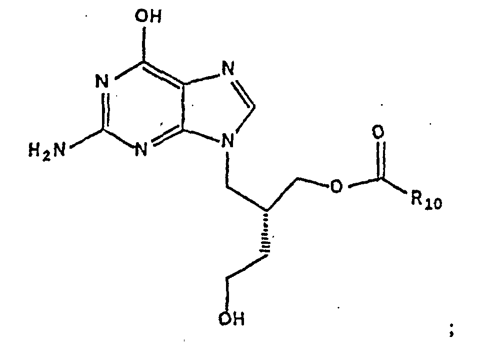 and
1c) reacting the product of step 1b) with P₁NHCH(R₁₁)COOH or an activated derivative thereof or with P₁NHCH(R₁₁)C(O)-O-C(O)CH(R)₁₁)NHP₁, wherein P₁ is an N-protecting group, or
2a) deprotecting the ether substituent of the compound of the formula: 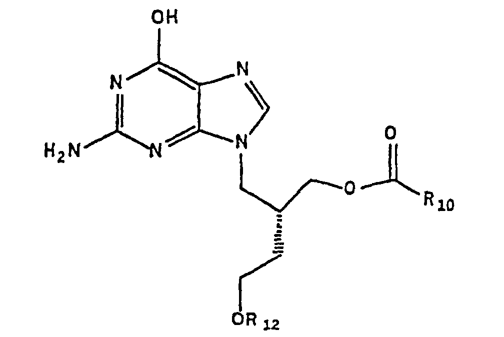 wherein R₁₂ is -CH(Ph)₂, -C(Ph)₃ or -Si(t-Bu)(CH₃)₂, wherein Ph is phenyl, to provide the alcohol of the formula: 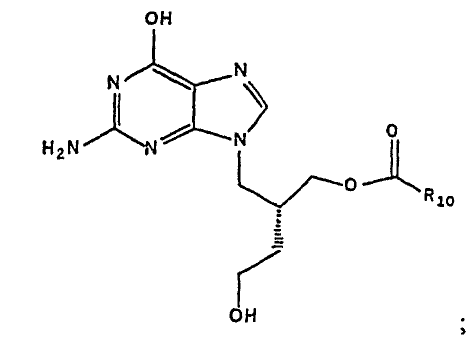 and
2b) reacting the product of step 2a) with P₁NHCH(R₁₁)COOH or an activated derivative thereof or with P₁NHCH(R₁₁)C(O)-O-C(O)CH(R₁₁)NHP₁, wherein R₁₁ and P₁ are as defined above, or
3) reacting a compound of the formula: 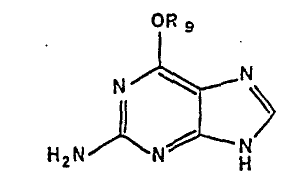
wherein R₉ is an alcohol protecting group with a compound of the formula: 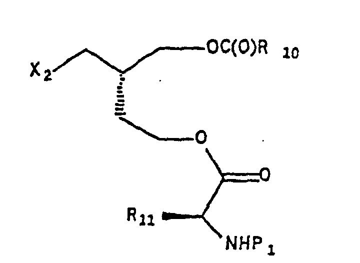 wherein X₂ is a halogen or sulfonate leaving group;
wherein the guanine moiety may also be in its ketomeric form.

15. The process of claim 14, the process further comprising N-deprotecting the product of steps 1c) and/or 2b).

16. The process of claim 14 or 15, wherein R₆ and R₇ are -CH₃ or -CH₂CH₃ or R₆ and R₇ taken together are -CH₂CH₂-, -CH₂CH₂CH₂- or -CH₂CH₂CH₂CH₂-, R₁₀ is C₉-C₁₉ alkyl, and P₁ is t-butyloxycarbonyl or benzyloxycarbonyl.

17. The process of claim 16, wherein R₆ and R₇ are -CH₂CH₃ and/or R₁₀ is -(CH₂)₁₆CH₃.

18. The process of claim 16 or 17, wherein R₉ is benzyl, R₁₀ is -(CH₂)₁₆CH₃, and X₂ is p-toluenesulfonyloxy.

19. The process of any of claims 14 to 18, wherein the acetal of step 1a) is deprotected by reaction with an acid or an acidic resin and/or wherein the aldehyde substituent of the product of step 1a) is reduced with borane t-butyl amine complex.

20. A compound of the formula: 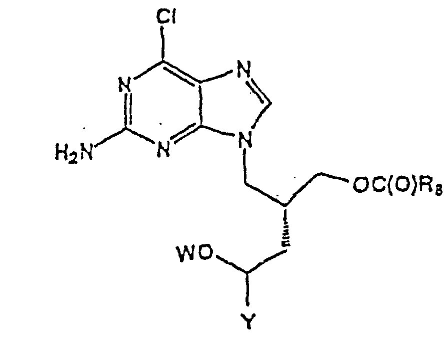 wherein Y is either -H or -OR₇, W is either R₆ or R₁₂, provided that W is R₁₂ if Y is -H, wherein R₆ and R₇ are C₁-C₇ alkyl or benzyl or R₆ and R₇ taken together are -CH₂CH₂-, -CH₂CH₂CH₂- or -CH₂CH₂CH₂CH₂-, R₈ is C₁-C₂₁ saturated or monounsaturated, optionally substituted alkyl, wherein the up to five similar or different substituents are independently selected from -OH, C₁-C₆ alkyl, C₁-C₆ alkoxy, C₁-C₆ alkoxy C₁-C₆ alkyl, C₁-C₆ alkanoyl, amino, halo, cyano, azido, oxo, mercapto, and nitro, and R₁₂ is -CH(Ph)₂, -C(Ph)₃ or -Si(t-Bu)(CH₃)₂ wherein Ph is phenyl.

21. The compound of claim 20, wherein R₈ is -CH₃ or -(CH₂)₁₆CH₃ and R₁₂ is as defined.

22. The compound of claim 20 or 21, wherein R₆ and R₇ are -CH₃ or -CH₂CH₃ or R₆ and R₇ taken together are -CH₂CH₂-, -CH₂CH₂CH₂- or -CH₂CH₂CH₂CH₂-, and R₈ is -CH₃.

23. The compound of any of claims 20 to 22, wherein R₆ and R₇ are -CH₂CH₃ and R₈ is -CH₃.

24. The compound of claim 20, wherein R₆ and R₇ are -CH₃ or -CH₂CH₃ or R₆ and R₇ taken together are -CH₂CH₂-, -CH₂CH₂CH₂- or -CH₂CH₂CH₂CH₂-, and R₈ is -(CH₂)₁₆CH₃, or wherein R₆ and R₇ are -CH₂CH₃ and R₈ is -(CH₂)₁₆CH₃.

25. A compound of the formula: 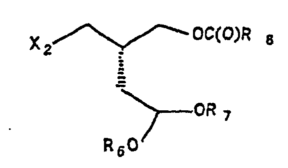 wherein R₆ and R₇ are C₁-C₇ alkyl or benzyl or R₆ and R₇ taken together are -CH₂CH₂-, -CH₂CH₂CH₂- or -CH₂CH₂CH₂CH₂-, R₈ is C₁-C₂₁ saturated or monounsaturated, optionally substituted alkyl, wherein the up to five similar or different substituents are independently selected from -OH, C₁-C₆ alkyl, C₁-C₆ alkoxy, C₁-C₆ alkoxy C₁-C₆ alkyl, C₁-C₆ alkanoyl, amino, halo, cyano, azido, oxo, mercapto, and nitro, and X₂ is a leaving group or -OH, provided that if R₆ and R₇ are -CH₂CH₃ and R₈ is -CH₃, then X₂ is not (a) -OH, (b) -O-C(=O)-C(CH₃)₃ or (c) -O-SO₂-CH₃.

26. The compound of claim 25, wherein R₈ is C₃-C₂₁ saturated or monounsaturated, optionally substituted alkyl, wherein the up to five similar or different substituents are independently selected from -OH, C₁-C₆ alkyl, C₁-C₆ alkoxy, C₁-C₆, alkoxy C₁-C₆ alkyl, C₁-C₆ alkanoyl, amino, halo, cyano, azido, oxo, mercapto, and nitro.

27. The compound of claims 25 or 26, wherein R₆ and R₇ are -CH₃ or -CH₂CH₃ or R₆ and R₇ taken together are -CH₂CH₂-, -CH₂CH₂CH₂- or -CH₂CH₂CH₂CH₂-, R₈ is -(CH₂)₁₆CH₃, and X₂ is a halogen, or a sulfonate leaving group, or -OH.

28. The compound of claim 27, wherein R₆ and R₇ are -CH₂CH₃, R₈ is -(CH₂)₁₆CH_{3,} and X₂ is tosylate or -OH.

29. The compound of claim 25, wherein the compound is (2S)-2-acetoxymethyl-4,4-diethoxybutyl toluenesulfonate.

30. A process for the preparation of the compound of any of claims 25 to 29, the process comprising the steps of
a) reacting a compound of the formula: 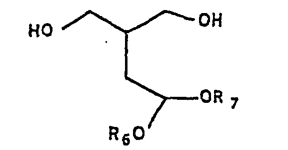 wherein R₆ and R₇ are as defined above, with CH₂=OH-OC(O)R₈, in the presence of a lipase to provide an ester of the formula: 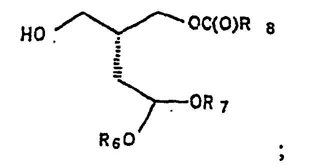 and optionally, if X₂ is desired to be a leaving group,
b) converting the hydroxyl group of the product of step a) to a leaving group.

31. A compound of the formula: 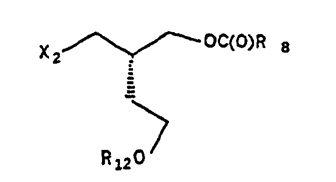 wherein R₈ is C₁-C₂₁ saturated or monounsaturated, optionally substituted alkyl, wherein the up to five similar or different substituents are independently selected from -OH, C₁-C₆ alkyl, C₁-C₆ alkoxy, C₁-C₆ alkoxy C₁-C₆ alkyl, C₁-C₆ alkanoyl, amino, halo, cyano, azido, oxo, mercapto, and nitro, R₁₂ is -CH(Ph)₂-C(Ph)₃, or -Si(t-Bu)(CH₃)₂, wherein Ph is phenyl, and X₂ is a leaving group or -OH, with the proviso that when R₈ is methyl and X₂ is -OH, then R₁₂ is not -Si(tert. butyl)(CH₃)₂.

32. The compound of claim 31, wherein R₈ is -(CH₂)₁₆CH₃ and X₂ is -OH.

33. The compound of claim 31, wherein R₈ is -(CH₂)₁₆CH₃ and X₂ is a halogen or a sulfonate leaving group.

34. The compound of claim 31, wherein R₈ is -(CH₂)₁₆CH₃ and X₂ is tosylate.

35. The compound of claim 31, wherein R₈ is -CH₃ and X₂ is -OH.

36. The compound of claim 31, wherein R₈ is -CH₃ and X₂ is a halogen or a sulfonate leaving group.

37. The compound of claim 31, wherein R₈ is -CH₃ and X₂ is tosylate.

38. A process for the preparation of the compound of any of claims 31 to 37, wherein R₈ is C₃-C₂₁ saturated or monounsaturated, optionally substituted alkyl, wherein the process comprises the steps of
a) reacting a compound of the formula: 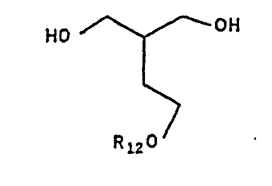 wherein R₁₂ is as defined above, with CH₂=CH-OC(O)R₈, wherein R₈ is as defined above, in the presence of a lipase to provide an ester of the formula: 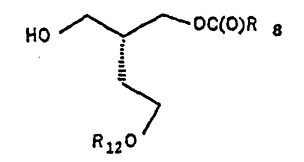 wherein R₈ and R₁₂ are as defined above; and optionally, if X₂ is desired to be a leaving group,
b) converting the hydroxyl group of the product of step a) to a leaving group.

39. A compound of the formula 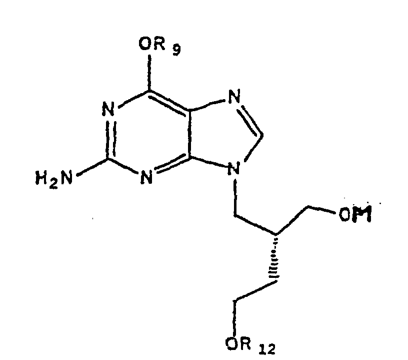 wherein R₉ is -H or an alcohol protecting group selected from substituted methyl ethers, tetrahydropyranyl ethers, substituted ethyl ethers, silyl ethers, and carboxylic acid esters, wherein if R₉ is -H the corresponding keto-tautomeric form is included; wherein R₁₂ is -CH(Ph)₂, -C(Ph)₃, or -Si(t-Bu) (CH₃)₂, wherein Ph is phenyl, M is -H or -C(O)R₁₀, R₁₀ being -C₃-C₂₁ saturated or monounsaturated, optionally substituted alkyl, wherein the up to five similar or different substituents are independently selected from -OH, C₁-C₆ alkyl, C₁-C₆ alkoxy, C₁-C₆ alkoxy C₁-C₆ alkyl, C₁-C₆ alkanoyl, amino, halo, cyano, azido, oxo, mercapto, and nitro, provided that
(a) M is -H if R₉ is an alcohol protecting group;
(b) R₁₂ is not -C(Ph)₃ if both M and R₉ are -H; and
(c) R₁₂ is not -C(Ph)₃ if M is -C(O)R₁₀ and R₉ is -H.

40. The compound of claim 39, wherein both R₉ and M are -H, R₉ is benzyl and M is -H, and/or R₁₀ is -(CH₂)₁₆CH₃ and R₉ is -H.

41. A compound of the formula: 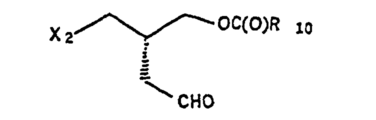 wherein X₂ is a halogen or sulfonate leaving group, R₁₀ is C₃-C₂₁ saturated or monounsaturated, optionally substituted alkyl, wherein the up to five similar or different substituents are independently selected from -OH, C₁-C₆ alkyl, C₁-C₆ alkoxy, C₁-C₆, alkoxy C₁-C₆ alkyl, C₁-C₆ alkanoyl, amino, halo, cyano, azido, oxo, mercapto, and nitro, M is -CHO or 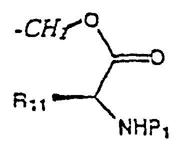 P₁ is an N-protecting group, and R₁₁ is isopropyl or isobutyl.

42. The compound of claim 41, wherein X₂ is p-toluenesulfonyloxy, R₁₀ is -(CH₂)₁₆CH₃, and L is -CHO or -CH₂OC(O)CHR₁₁NHP₁, with R₁₁ being isopropyl and P₁ being benzyloxycarbonyl.

## Patentansprüche

1. Verbindung der Formel: 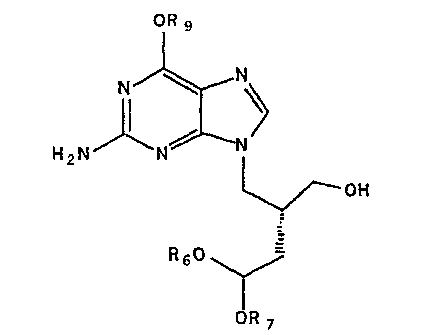 in der R₆ und R₇ C₁-C₇-Alkyl oder Benzyl sind, oder in der R₆ und R₇ zusammen -CH₂CH₂-, -CH₂CH₂CH₂- oder -CH₂CH₂CH₂CH₂- sind, und in der R₉ -H oder eine Alkohol-Schutzgruppe, ausgewählt aus substituierten Methylethem, Tetrahydropyranylethern, substituierten Ethylethern, Silylethern und Carbonsäureestern, ist, und wobei, falls R₉ -H ist, die entsprechende keto-tautomere Form umfasst ist.

2. Verbindung nach Anspruch 1, wobei der substituierte Methylether Methoxymethyl, Benzyloxymethyl, 2-(Trimethylsilyl-)ethoxymethyl, t-Butyl, Benzyl oder Triphenylmethyl ist; wobei der substituierte Ethylether 2,2,2-Trichlorethyl ist; wobei der Silylether Trimethylsilyl, t-Butyldimethylsilyl oder t-Butyldiphenylsilyl ist; und wobei der Carbonsäureester Acetat, Propionat oder Benzoat ist.

3. Verbindung nach Anspruch 1 oder 2, wobei R₆ und R₇ -CH₃ oder -CH₂CH₃ sind, oder wobei R₆ und R₇ zusammen -CH₂CH₂-, -CH₂CH₂CH₂- oder -CH₂CH₂CH₂CH₂- sind, und wobei R₉ Benzyl ist.

4. Verbindung nach Anspruch 3, wobei R₆ und R₇ -CH₂CH₃ sind und R₉ Benzyl ist.

5. Verbindung nach Anspruch 1, wobei R₆ und R₇ -CH₂CH₃ sind, wobei R₉ -H ist und wobei die entsprechende keto-tautomere Form umfasst ist.

6. Verfahren für die Herstellung der Verbindung nach einem der vorhergehenden Ansprüche, wobei das Verfahren den Schritt umfasst, eine Verbindung der Formel 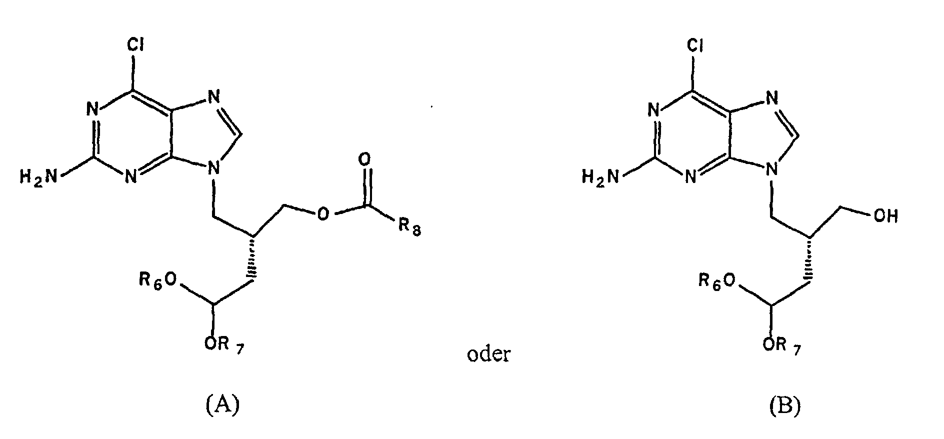 wobei R₆ und R₇ wie oben definiert sind und R₈ C₁-C₂₁ gesättigtes oder einfach ungesättigtes, ggf. substituiertes Alkyl ist, in Gegenwart einer Base, die Kalium-t-butoxid, Kaliumcarbonat, NaH, KH oder Lithium-diisopropylamid ist, mit einer anorganischen Base oder, falls R₉ eine Alkohol-Schutzgruppe ist, mit einem Alkohol R₉OH umzusetzen, wobei das Verfahren ggf. den weiteren sich anschließenden Schritt der Abspaltung von R₉ unter Erhalt des entsprechenden Alkohols umfasst.

7. Verfahren nach Anspruch 6, wobei R₆ und R₇ in der Verbindung der Formel (A) -CH₂CH₃ sind, R₈ Methyl ist und das Verfahren den Schritt umfasst, die Verbindung der Formel (A) mit Kaliumcarbonat umzusetzen.

8. Verfahren nach Anspruch 6, wobei die anorganische Base KOH oder NaOH ist.

9. Verbindung der Formel 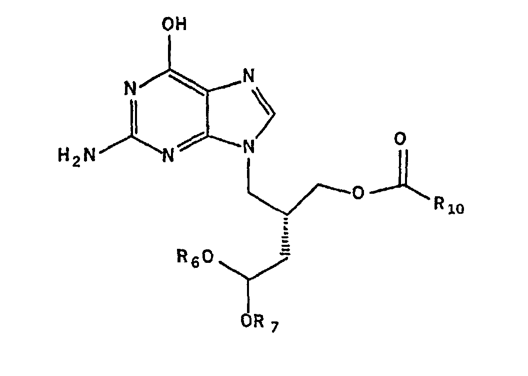 in der R₆ und R₇ C₁-C₇-Alkyl oder Benzyl sind, oder in der R₆ und R₇ zusammen -CH₂CH₂-, -CH₂CH₂CH₂- oder -CH₂CH₂CH₂CH₂- sind, und in der R₁₀ C₃-C₂₁ gesättigtes oder einfach ungesättigtes, ggf. substituiertes Alkyl ist, wobei die bis zu 5 ähnlichen oder verschiedenen Substituenten unabhängig voneinander ausgewählt sind aus -OH, C₁-C₆ Alkyl, C₁-C₆ Alkoxy, C₁-C₆ Alkoxy C₁-C₆ Alkyl, C₁-C₆ Alkanoyl, Amino, Halo, Cyano, Azido, Oxo, Mercapto und Nitro; und deren Keto-Tautomere.

10. Verbindung nach Anspruch 9, wobei R₆ und R₇ -CH₃ oder -CH₂CH₃ sind, oder wobei R₆ und R₇ zusammen -CH₂CH₂-, -CH₂CH₂CH₂- oder -CH₂CH₂CH₂CH₂- sind und R₁₀ -(CH₂)₁₆CH₃ ist.

11. Verbindung nach Anspruch 10, wobei R₆ und R₇ -CH₂CH₃ sind, und/oder wobei R₁₀ -(CH₂)₁₆CH₃ ist.

12. Verfahren für die Herstellung der Verbindung nach einem der Ansprüche 9 bis 11, wobei das Verfahren den Schritt umfasst, eine Verbindung der Formel 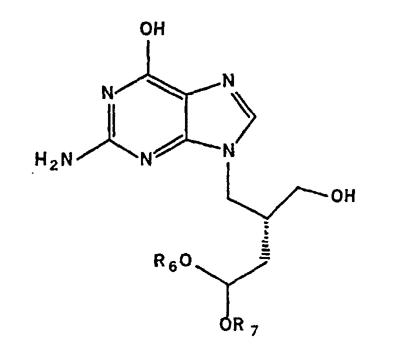 oder ihr Keto-Tautomeres, wobei R₆ und R₇ wie oben definiert sind, mit R₁₀COOH oder mit einem seiner aktivierten Derivate umzusetzen.

13. Verfahren nach Anspruch 12, wobei das aktivierte Derivat von R₁₀COOH R₁₀C(O)OS(O)₂R₃₀, wobei R₃₀ C₁-C₇ Alkyl, Phenyl or Toluyl ist, R₁₀C(O)OC(O)R₁₀ oder R₁₀C(O)OC(O)R₁₀ₐ, wobei R₁₀ₐ C₁-C₇ Alkyl ist und wobei R₁₀ -(CH₂)₁₆CH₃ ist.

14. Verfahren für die Herstellung einer Verbindung der Formel 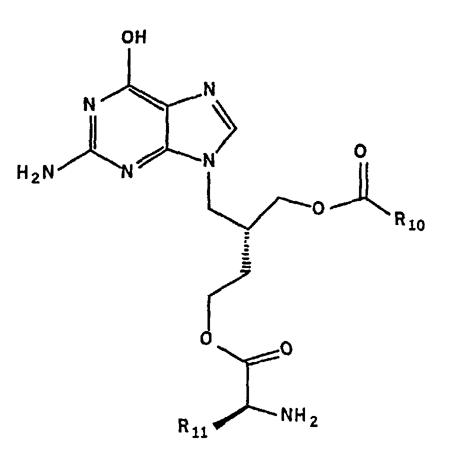 wobei R₁₀ C₃-C₂₁ gesättigtes oder einfach ungesättigtes, ggf. substituiertes Alkyl ist, R₁₁ Isopropyl or Isobutyl ist und das Verfahren eine der drei folgenden Abfolgen von Schritten umfasst:
1a) das Acetal als Schutzgruppe in der Verbindung der Formel: 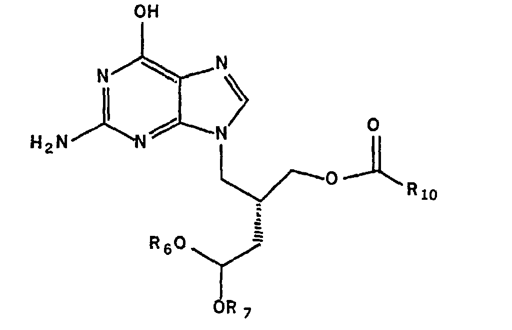 in der R₆ und R₇ C₁-C₇ Alkyl oder Benzyl sind, oder in der R₆ and R₇ zusammen -CH₂CH₂-, -CH₂CH₂CH₂- or -CH₂CH₂CH₂CH₂- sind, abzuspalten; und
1b) den Aldehyd-Substituenten des Produkts von Schritt 1a) unter Herstellung des Alkohols der Formel: 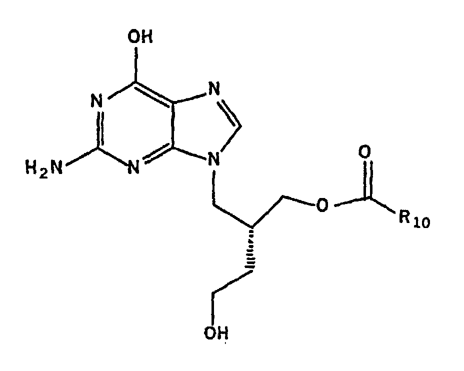 zu reduzieren; und
1c) das Produkt von Schritt 1b) mit with P₁NHCH(R₁₁)COOH, mit einem aktivierten Derivat davon oder mit P₁NHCH(R₁₁)C(O)-O-C(O)CH(R₁₁)NHP₁, wobei P₁ eine N-Schutzgruppe ist, umzusetzen, oder
2a) den Ether-Substituenten als Schutzgruppe in der Verbindung der Formel: 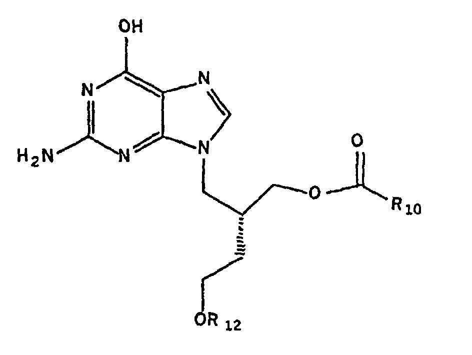 wobei R₁₂ -CH(Ph)₂, -C(Ph)₃ oder -Si(t-Bu)(CH₃)₂ ist, wobei Ph Phenyl ist, unter Herstellung des Alkohols der Formel: 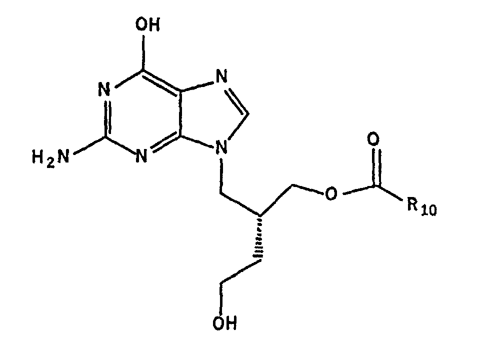 abzuspalten; und
2b) das Produkt von Schritt 2a) mit P₁NHCH(R₁₁)COOH, mit einem aktivierten Derivat davon oder mit P₁NHCH(R₁₁)C(O)-O-C(O)CH(R₁₁)NHP₁, wobei R₁₁ und P₁ wie oben definiert sind, umzusetzen, oder
3) eine Verbindung der Formel: 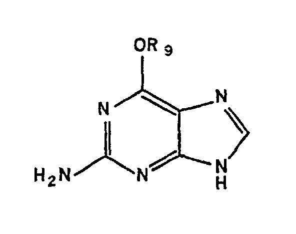 in der R₉ eine Alkohol-Schutzgruppe ist, mit einer Verbindung der Formel: 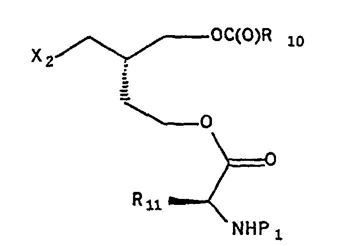 in der X₂ ein Halogen oder eine Sulfonat-Abgangsgruppe ist, umzusetzen;
wobei der Guanin-Rest auch in seiner Ketoform vorliegen kann.

15. Verfahren nach Anspruch 14, wobei das Verfahren außerdem den Schritt umfasst, die N-Schutzgruppen der Produkte der Schritte 1c) und/oder 2b) abzuspalten.

16. Verfahren nach Anspruch 14 oder 15, wobei R₆ und R₇ -CH₃ oder -CH₂CH₃ sind, oder wobei R₆ and R₇ zusammen -CH₂CH₂-, -CH₂CH₂CH₂- oder -CH₂CH₂CH₂CH₂- sind, R₁₀ C₉-C₁₉ Alkyl ist und P₁ t-Butyloxycarbonyl oder Benzyloxycarbonyl ist.

17. Verfahren nach Anspruch 16, wobei R₆ und R₇ -CH₂CH₃ sind und/oder wobei R₁₀ -(CH₂)₁₆CH₃ ist.

18. Verfahren nach Anspruch 16 oder 17, wobei R₉ Benzyl ist, R₁₀ -(CH₂)₁₆CH₃ ist und X₂ p-Toluolsulfonyloxy ist.

19. Verfahren nach einem der Ansprüche 14 bis 18, wobei das Acetal als Schutzgruppe des Produkts von Schritt 1a) mittels Reaktion mit einer Säure oder einem sauren Harz abgespalten wird und/oder wobei der Aldehyd-Substituent des Produkts von Schritt 1a) mit Boran-t-butylamin-Komplex reduziert wird.

20. Verbindung der Formel 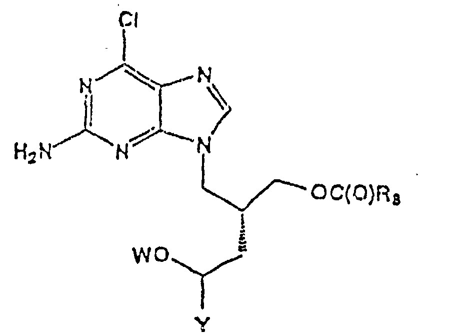 wobei Y entweder -H oder -OR₇ ist, wobei W entweder R₆ oder R₁₂ ist, vorausgesetzt W ist R₁₂, wenn Y -H ist, wobei R₆ und R₇ C₁-C₇ Alkyl oder Benzyl sind, oder wobei R₆ und R₇ zusammen -CH₂CH₂-, -CH₂CH₂CH₂- oder -CH₂CH₂CH₂CH₂- sind, wobei R₈ C₁-C₂₁ gesättigtes oder einfach ungesättigtes, ggf. substituiertes Alkyl ist, wobei die bis zu 5 ähnlichen oder verschiedenen Substituenten unabhängig voneinander ausgewählt sind aus -OH, C₁-C₆ Alkyl, C₁-C₆ Alkoxy, C₁-C₆ Alkoxy C₁-C₆ Alkyl, C₁-C₆ Alkanoyl, Amino, Halo, Cyano, Azido, Oxo, Mercapto und Nitro, und wobei R₁₂ -CH(Ph)₂, -C(Ph)₃ oder -Si(t-Bu)(CH₃)₂, wobei Ph Phenyl ist, ist.

21. Verbindung nach Anspruch 20, wobei R₈ -CH₃ oder -(CH₂)₁₆CH₃ ist und R₁₂ wie definiert ist.

22. Verbindung nach Anspruch 20 oder 21, wobei R₆ und R₇ -CH₃ oder -CH₂CH₃ sind, oder wobei R₆ and R₇ zusammen -CH₂CH₂-, -CH₂CH₂CH₂- oder -CH₂CH₂CH₂CH₂- sind und R₈ -CH₃ ist.

23. Verbindung nach einem der Ansprüche 20 bis 22, wobei R₆ und R₇ -CH₂CH₃ sind und R₈ -CH₃ ist.

24. Verbindung nach Anspruch 20, wobei R₆ und R₇ -CH₃ oder -CH₂CH₃ sind, oder wobei R₆ und R₇ zusammen -CH₂CH₂-, -CH₂CH₂CH₂- oder -CH₂CH₂CH₂CH₂- sind und R₈ -(CH₂)₁₆-CH₃ ist, oder wobei R₆ und R₇ -CH₂CH₃ sind und R₈ -(CH₂)₁₆CH₃ ist.

25. Verbindung der Formel: 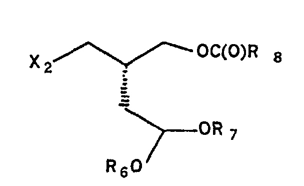 wobei R₆ und R₇ C₁-C₇ Alkyl oder Benzyl sind, oder wobei R₆ und R₇ zusammen -CH₂CH₂-, -CH₂CH₂CH₂- oder -CH₂CH₂CH₂CH₂- sind, R₈ C₁-C₂₁ gesättigtes oder einfach ungesättigtes, ggf. substituiertes Alkyl ist, wobei die bis zu 5 ähnlichen oder verschiedenen Substituenten unabhängig voneinander ausgewählt sind aus -OH, C₁-C₆ Alkyl, C₁-C₆ Alkoxy, C₁-C₆ Alkoxy C₁-C₆ Alkyl, C₁-C₆ Alkanoyl, Amino, Halo, Cyano, Azido, Oxo, Mercapto, und Nitro, und wobei X₂ eine Abgangsgruppe oder -OH ist, vorausgesetzt X₂ ist nicht (a) -OH, (b) -O-C(=O)-C(CH₃)₃ oder (c) -O-SO₂-CH₃, wenn R₆ und R₇ -CH₂CH₃ sind und R₈ -CH₃ ist.

26. Verbindung nach Anspruch 25, wobei R₈ C₃-C₂₁ gesättigtes oder einfach ungesättigtes, ggf. substituiertes Alkyl ist, wobei die bis zu 5 ähnlichen oder verschiedenen Substituenten unabhängig voneinander ausgewählt sind aus -OH, C₁-C₆ Alkyl, C₁-C₆ Alkoxy, C₁-C₆ Alkoxy C₁-C₆ Alkyl, C₁-C₆ Alkanoyl, Amino, Halo, Cyano, Azido, Oxo, Mercapto und Nitro.

27. Verbindung nach Anspruch 25 oder 26, wobei R₆ und R₇ -CH₃ oder -CH₂CH₃ sind, oder wobei R₆ und R₇ zusammen -CH₂CH₂-, -CH₂CH₂CH₂- oder -CH₂CH₂CH₂CH₂- sind, R₈ -(CH₂)₁₆CH₃ ist und X₂ ein Halogen, eine Sulfonat-Abgangsgruppe oder -OH ist.

28. Verbindung nach Anspruch 27, wobei R₆ und R₇ -CH₂CH₃ sind, R₈ -(CH₂)₁₆CH₃ ist und X₂ Tosylat oder -OH ist.

29. Verbindung nach Anspruch 25, wobei die Verbindung (2S)-2-Acetoxymethyl-4,4-diethoxybutyl-toluolsulfonat ist.

30. Verfahren für die Herstellung der Verbindung nach einem der Ansprüche 25 bis 29, wobei das Verfahren die Schritte umfasst,
a) eine Verbindung der Formel: 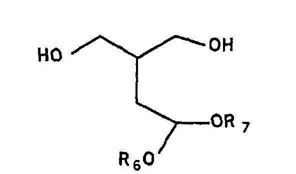 in der R₆ und R₇ wie oben definiert sind, in Gegenwart einer Lipase mit CH₂=CH-OC(O)R₈ zur Herstellung eines Esters der Formel: 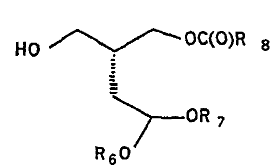 umzusetzen, und ggf., wenn X₂ eine Abgangsgruppe sein soll,
b) die Hydroxyl-Gruppe des Produkts von Schritt a) in eine Abgangsgruppe umzuwandeln.

31. Verbindung der Formel: 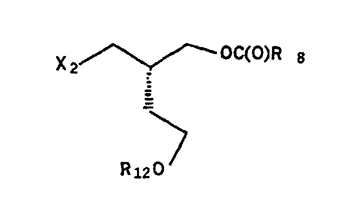 in der R₈ C₁-C₂₁ gesättigtes oder einfach ungesättigtes, ggf. substituiert es Alkyl ist, wobei die bis zu 5 ähnlichen oder verschiedenen Substituenten unabhängig voneinander ausgewählt sind aus -OH, C₁-C₆ Alkyl, C₁-C₆ Alkoxy, C₁-C₆ Alkoxy C₁-C₆ Alkyl, C₁-C₆ Alkanoyl, Amino, Halo, Cyano, Azido, Oxo, Mercapto und Nitro, wobei R₁₂ -CH(Ph)₂-C(Ph)₃ oder -Si(t-Bu)(CH₃)₂, wobei Ph Phenyl ist, ist, und wobei X₂ eine Abgangsgruppe oder -OH ist, unter der Voraussetzung, dass R₁₂ nicht -Si(t-butyl)(CH₃)₂ ist, wenn R₈ Ethyl ist und X₂ -OH ist.

32. Verbindung nach Anspruch 31, wobei R₈ -(CH₂)₁₆CH₃ ist und X₂ -OH ist.

33. Verbindung nach Anspruch 31, wobei R₈ -(CH₂)₁₆CH₃ ist und X₂ ein Halogen oder eine Sulfonat-Abgangsgruppe ist.

34. Verbindung nach Anspruch 31, wobei R₈ -(CH₂)₁₆CH₃ ist und X₂ Tosylat ist.

35. Verbindung nach Anspruch 31, wobei R₈ -CH₃ ist und X₂ -OH ist.

36. Verbindung nach Anspruch 31, wobei R₈ -CH₃ ist und X₂ ein Halogen oder eine Sulfonat-Abgangsgruppe ist.

37. Verbindung nach Anspruch 31, wobei R₈ -CH₃ ist und X₂ Tosylat ist.

38. Verfahren für die Herstellung der Verbindung nach einem der Ansprüche 31 bis 37, wobei R₈ C₃-C₂₁ gesättigtes oder einfach ungesättigtes, ggf. substituiertes Alkyl ist, wobei das Verfahren die Schritte umfasst,
a) eine Verbindung der Formel: 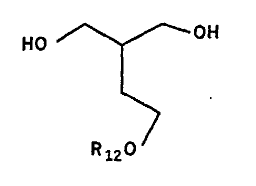 in der R₁₂ wie oben definiert ist, mit CH₂=CH-OC(O)R₈, wobei R₈ wie oben definiert ist, in Gegenwart einer Lipase unter Herstellung eines Esters der Formel: 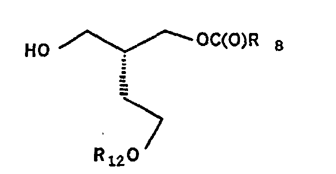 in der R₈ and R₁₂ wie oben definiert sind, umzusetzen; und ggf., wenn X₂ eine Abgangsgruppe sein soll,
b) die Hydroxyl-Gruppe des Produkts von Schritt a) in eine Abgangsgruppe umzuwandeln.

39. Verbindung der Formel: 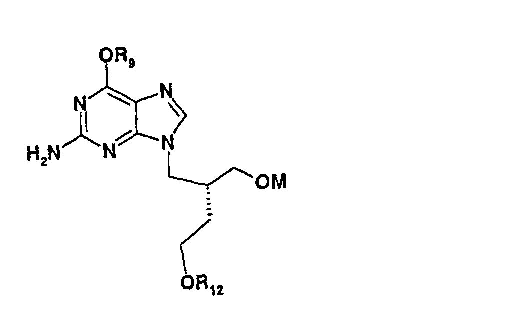 in der R₉ -H oder eine Alkohol-Schutzgruppe ist, ausgewählt aus substituierten Methylethern, Tetrahydropyranylethern, substituierten Ethylethern, Silylethem und Carbonsäureestern, wobei das entsprechende Keto-Tautomere umfasst ist, wenn R₉ -H ist; in der R₁₂ -CH(Ph)₂, -C(Ph)₃ oder -Si(t-Bu)(CH₃)₂ ist, wobei Ph Phenyl ist, wobei M -H oder -C(O)R₁₀ ist, wobei R₁₀ C₃-C₂₁ gesättigtes oder einfach ungesättigtes, ggf. substituiertes Alkyl ist, wobei die bis zu 5 ähnlichen oder verschiedenen Substituenten unabhängig voneinander ausgewählt sind aus -OH, C₁-C₆ Alkyl, C₁-C₆ Alkoxy, C₁-C₆ Alkoxy C₁-C₆ Alkyl, C₁-C₆ Alkanoyl, Amino, Halo, Cyano, Azido, Oxo, Mercapto und Nitro, vorausgesetzt
(a) M ist -H, wenn R₉ eine Alkohol-Schutzgruppe ist;
(b) R₁₂ ist nicht -C(Ph)_{3,} wenn sowohl M als auch R₉ -H sind; und
(c) R₁₂ ist nicht -C(Ph)₃, wenn M -C(O)R₁₀ ist und R₉ -H ist.

40. Verbindung nach Anspruch 39, wobei sowohl R₉ als auch M -H sind, R₉ Benzyl ist und M -H ist, und/oder wobei R₁₀ -(CH₂)₁₆CH₃ ist und R₉ -H ist.

41. Verbindung der Formel: 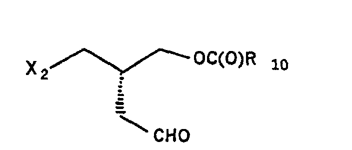 in der X₂ ein Halogen oder eine Sulfonat-Abgangsgruppe ist, in der R₁₀ C₃-C₂₁ gesättigtes oder ungesättigtes, ggf. substituiertes Alkyl ist, wobei die bis zu 5 ähnlichen oder verschiedenen Substituenten unabhängig voneinander ausgewählt sind aus -OH, C₁-C₆ Alkyl, C₁-C₆ Alkoxy, C₁-C₆ Alkoxy C₁-C₆ Alkyl, C₁-C₆ Alkanoyl, Amino, Halo, Cyano, Azido, Oxo, Mercapto und Nitro, in der M -CHO oder 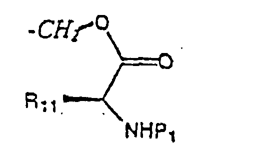 ist, in der P₁ eine N-Schutzgruppe ist, und in der R₁ Isopropyl oder Isobutyl ist.

42. Verbindung nach Anspruch 41, wobei X₂ p-Toluolsulfonyloxy ist, R₁₀ -(CH₂)₁₆CH₃ ist und L -CHO oder -CH₂OC(O)CHR₁₁NHP₁ ist, wobei R₁₁ Isopropyl ist und P₁ Benzyloxycarbonyl ist.

## Revendications

1. Composé de formule : 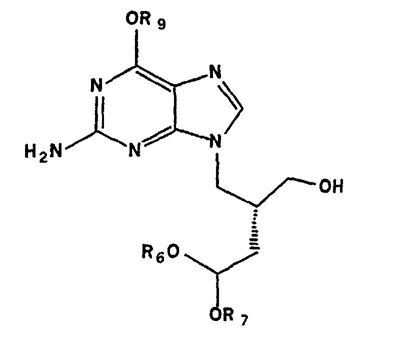 dans laquelle R₆ et R₇ sont (C₁-C₇)-alkyle ou benzyle ou R₆ et R₇ pris ensemble sont -CH₂CH₂-, -CH₂CH₂CH₂- ou -CH₂CH₂CH₂CH₂- et R₉ est -H ou un groupe protecteur de la fonction alcool choisi parmi les éthers méthyliques substitués, les éthers de tétrahydropyranyle, les éthers éthyliques substitués, les éthers de silyle et les esters d'acide carboxylique; dans laquelle lorsque R₉ est -H la forme correspondante tautomère céto est incluse.

2. Composé selon la revendication 1, dans laquelle l'éther méthylique substitué est le méthoxyméthyle, benzyloxyméthyle, 2-méthoxyéthoxyméthyle, 2-(triméthylsilyl)éthoxyméthyle, tert-butyle, benzyle ou triphénylméthyle; dans laquelle l'éther éthylique substitué est le 2,2,2-trichloroéthyle ; dans laquelle l'éther de silyle est le triméthylsilyle, tert-butyldiméthylsilyle ou tert-butydiphénylsilyle ; et dans laquelle l'ester d'acide carboxylique est l'acétate, proprionate, ou benzoate.

3. Composé selon la revendication 1 ou 2, dans laquelle R₆ et R₇ sont -CH₃ ou -CH₂CH₃ ou R₆ et R₇ pris ensemble sont -CH₂CH₂-, -CH₂CH₂CH₂- ou -CH₂CH₂CH₂CH₂- et R₉ est benzyle.

4. Composé selon la revendication 3, dans laquelle R₆ et R₇ sont -CH₂CH₃ et R₉ est benzyle.

5. Composé selon la revendication 1, dans laquelle R₆ et R₇ sont -CH₂CH₃ et R₉ est -H, la forme tautomère céto correspondante étant incluse.

6. Procédé de préparation du composé selon l'une quelconque des revendications précédentes, le procédé comprenant l'étape de réaction d'un composé de formule : 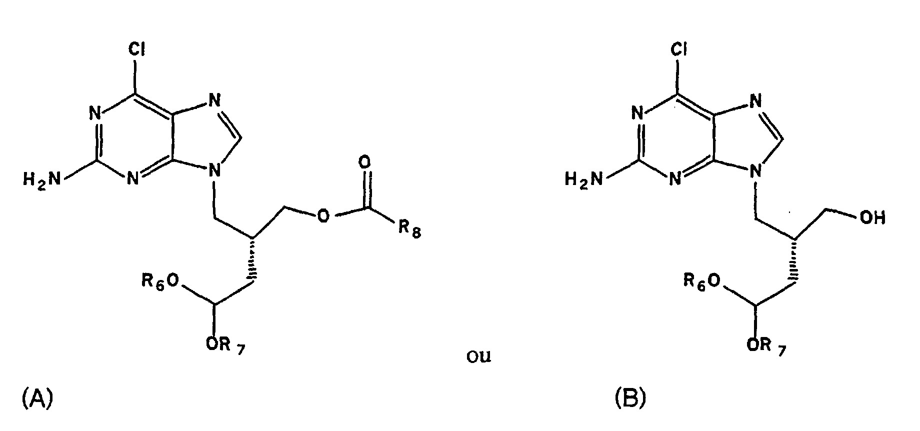 dans lesquelles R₆ et R₇ sont tels que définis ci-dessus et R₈ est (C₁-C₂₁)-alkyle saturé ou mono-insaturé, éventuellement substitué, avec une base inorganique ou, dans le cas où R₉ est un groupe protecteur de la fonction alcool, avec un alcool R₉OH en présence d'une base qui est le tert-butylate de potassium, le carbonate de potassium, NaH, KH, ou le diisopropylamide de lithium, éventuellement suivie par la déprotection de R₉ en l'alcool correspondant.

7. Procédé selon la revendication 6, dans laquelle dans le composé de formule (A), R₆ et R₇ sont -CH₂CH₃ et R₈ est méthyle, le procédé comprenant l'étape de réaction du composé de formule (A) avec le carbonate de potassium.

8. Procédé selon la revendication 6, dans laquelle la base inorganique est KOH ou NaOH.

9. Composé de formule : 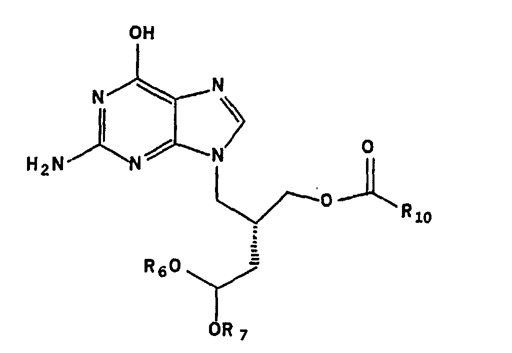 dans laquelle R₆ et R₇ sont (C₁-C₇)-alkyle ou benzyle ou R₆ et R₇ pris ensemble sont -CH₂CH₂-, -CH₂CH₂CH₂- ou -CH₂CH₂CH₂CH₂- et R₁₀ est un groupe (C₃-C₂₁)-alkyle saturé ou mono-insaturé, éventuellement substitué ; dans lequel les jusqu'à cinq substituants identiques ou différents sont indépendamment choisis parmi -OH, (C₁-C₆)-alkyle, (C₁-C₆)-alkoxy, (C₁-C₆)-alkoxy (C₁-C₆)-alkyle, (C₁-C₆)-alkanoyle, amino, halogèno, cyano, azido, oxo, mercapto, et nitro ; et les formes tautomères cétomères de ces composés.

10. Composé selon la revendication 9, dans laquelle R₆ et R₇ sont -CH₃ ou -CH₂CH₃ ou R₆ et R₇ pris ensemble sont -CH₂CH₂-, -CH₂CH₂CH₂- ou -CH₂CH₂CH₂CH₂- et R₁₀ est -(CH₂)₁₆CH₃.

11. Composé selon la revendication 10, dans laquelle R₆ et R₇ sont -CH₂CH₃ et/ou R₁₀ est -(CH₂)₁₆CH₃.

12. Procédé de préparation du composé selon l'une quelconque des revendications 9 à 11, le procédé comprenant l'étape de réaction d'un composé de formule : 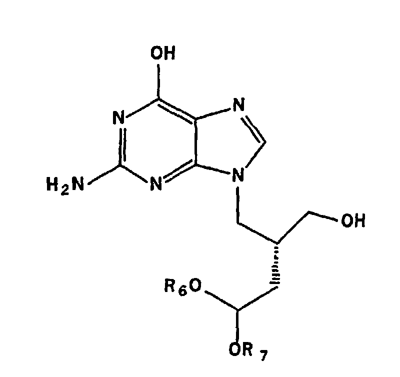 ou son tautomère cétomère, dans laquelle R₆ et R₇ sont tels que définis ci-dessus avec R₁₀COOH ou un dérivé activé de celui-ci.

13. Procédé selon la revendication 12, dans laquelle, le dérivé activé de R₁₀COOH est R₁₀C(O)OS(O)₂R₃₀, dans laquelle R₃₀ est (C₁-C₇)-alkyle, phényle ou toluyle, ou R₁₀C(O)OC(O)R₁₀ ou R₁₀C(O)OC(O)R_{10a,} dans laquelle R₁₀ₐ est (C₁-C₇)-alkyle, et dans laquelle R₁₀ est -(CH₂)₁₆CH₃.

14. Procédé de préparation d'un composé de formule : 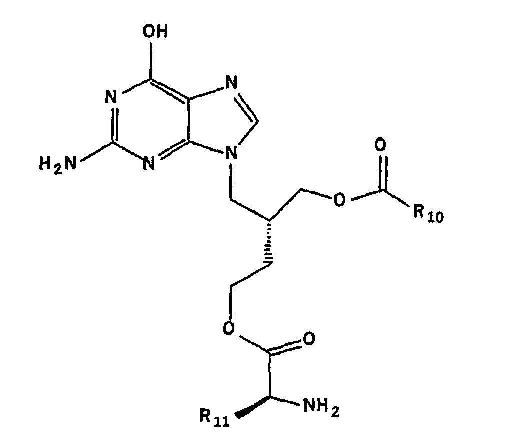 dans laquelle R₁₀ est un groupe (C₃-C₂₁)-alkyle saturé ou mono-insaturé, éventuellement substitué et R₁₁ est isopropyle ou isobutyle, le procédé comprenant une des trois séquences d'étapes suivantes :
1a) déprotection de l'acétal du composé de formule : 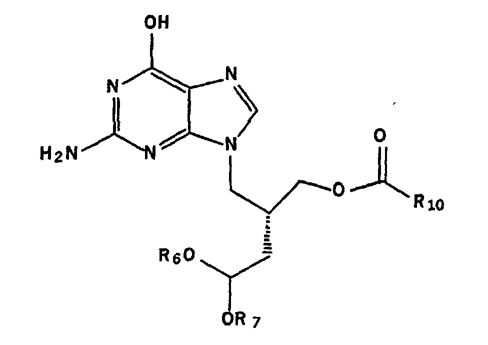 dans laquelle R₆ et R₇ sont (C₁-C₇)-alkyle ou benzyle ou R₆ et R₇ pris ensemble sont -CH₂CH₂-, -CH₂CH₂CH₂- ou -CH₂CH₂CH₂CH₂- ; et
1b) réduction du substituant aldéhyde du produit de l'étape 1a) pour fournir l'alcool de formule : 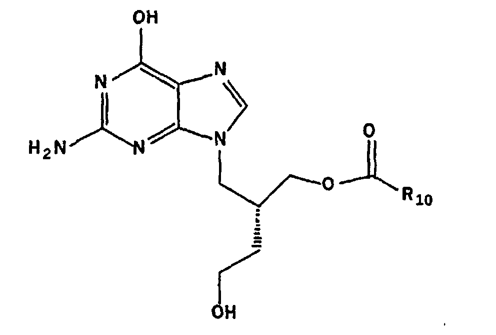 et
1c) réaction du produit de l'étape 1b) avec P₁NHCH(R₁₁)COOH ou un dérivé activé de celui-ci ou avec P₁NHCH(R₁₁)C(O)-O-C(O)CH(R₁₁)NHP₁, dans laquelle P₁ est un groupe N-protecteur, ou
2a) déprotection du substituant éther du composé de formule : 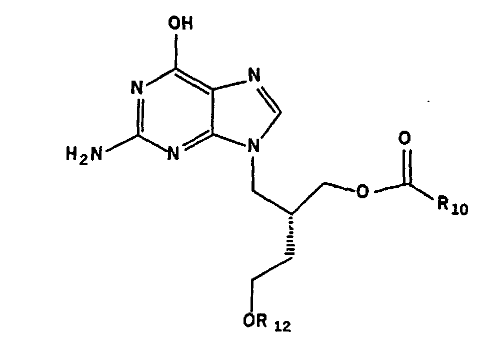 dans laquelle R₁₂ est -CH(Ph)₂, -C(Ph)₃ ou Si(tert-Bu)(CH₃)_{2,} dans laquelle Ph est phényle, pour fournir l'alcool de formule : 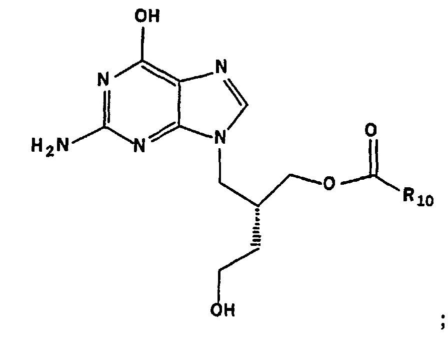 et
2b) réaction du produit de l'étape 2a) avec P₁NHCH(R₁₁)COOH ou un dérivé activé de celui-ci ou avec P₁NHCH(R₁₁)C(O)-O-C(O)CH(R₁₁)NHP₁, dans laquelle R₁₁ et P₁ sont tels que définis ci-dessus, ou
3) réaction d'un composé de formule : 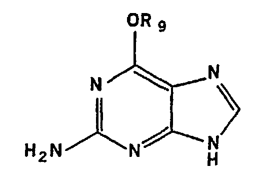
dans laquelle R₉ est un groupe protecteur de la fonction alcool avec un composé de la formule : 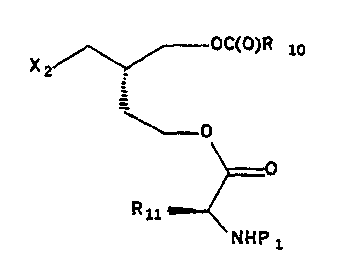 dans laquelle X₂ est halogène ou un groupe partant sulfonate ;
dans laquelle le reste guanine peut également être sous sa forme cétomère.

15. Procédé selon la revendication 14, le procédé comprenant en outre la N-déprotection du produit des étapes 1c) et/ou 2b).

16. Procédé selon la revendication 14 ou 15, dans laquelle R₆ et R₇ sont -CH₃ ou -CH₂CH₃ ou R₆ et R₇ pris ensemble sont -CH₂CH₂-, -CH₂CH₂CH₂- ou -CH₂CH₂CH₂CH₂- et R₁₀ est (C₉-C₁₉)-alkyle, et P₁ est tert-butyloxylcarbonyle ou benzyloxycarbonyle.

17. Procédé selon la revendication 16, dans laquelle R₆ et R₇ sont -CH₂CH₃ et/ou R₁₀ est -(CH₂)₁₆CH₃.

18. Procédé selon la revendication 16 ou 17, dans laquelle R₉ est benzyle, R₁₀ est -(CH₂)₁₆CH₃, et X₂ est p-toluènesylfonyloxy.

19. Procédé selon l'une des revendications 14 à 18, dans laquelle l'acétal de l'étape 1a) est déprotégée par réaction avec un acide ou une résine acide et/ou dans laquelle le substituant aldéhyde du produit de l'étape 1a) est réduit avec un complexe de tert-butylamine borane.

20. Composé de formule : 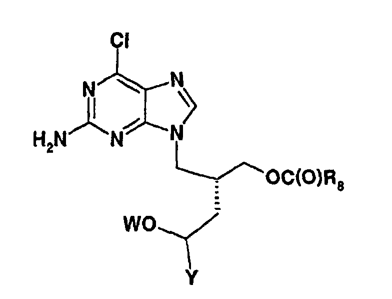 dans laquelle Y est soit -H soit -OR₇, W est soit R₆ soit R₁₂ sous réserve que W est R₁₂ lorsque Y est -H, dans laquelle R₆ et R₇ sont (C₁-C₇)-alkyle ou benzyle ou R₆ et R₇ pris ensemble sont -CH₂CH₂-, -CH₂CH₂CH₂- ou -CH₂CH₂CH₂CH₂- et R₈ est (C₁-C₂₁)-alkyle saturé ou mono-insaturé, éventuellement substitué, dans lequel les jusqu'à cinq substituants identiques ou différents sont indépendamment choisis parmi -OH, (C₁-C₆)-alkyle, (C₁-C₆)-alkoxy, (C₁-C₆)-alkoxy (C₁-C₆)-alkyle, (C₁-C₆)-alkanoyle, amino, halogèno, cyano, azido, oxo, mercapto, et nitro ; et R₁₂ est -CH(Ph)₂, -C(Ph)₃ ou Si(tert-Bu)(CH₃)_{2,} où Ph est phényle.

21. Composé selon la revendication 20, dans laquelle R₈ est -CH₃ ou -(CH₂)₁₆CH₃ et R₁₂ est tel que défini.

22. Composé selon la revendication 20 ou 21, dans laquelle R₆ et R₇ sont -CH₃ ou -CH₂CH₃ ou R₆ et R₇ pris ensemble sont -CH₂CH₂-, -CH₂CH₂CH₂- ou -CH₂CH₂CH₂CH₂- et R₈ est -CH₃.

23. Composé selon l'une quelconque des revendications 20 à 22, dans laquelle R₆ et R₇ sont -CH₂CH₃ et R₈ est -CH₃.

24. Composé selon la revendication 20, dans laquelle R₆ et R₇ sont -CH₃ ou -CH₂CH₃ ou R₆ et R₇ pris ensemble sont -CH₂CH₂-, -CH₂CH₂CH₂- ou -CH₂CH₂CH₂CH₂- et R₈ est -(CH₂)₁₆CH₃ ou dans laquelle R₆ et R₇ sont -CH₂CH₃ et R₈ est -(CH₂)₁₆CH₃.

25. Composé de formule : 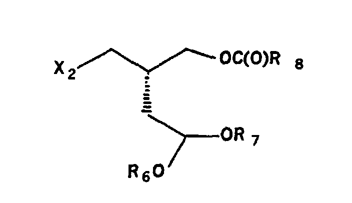 dans laquelle R₆ and R₇ sont (C₁-C₇)-alkyle ou benzyle ou R₆ et R₇ pris ensemble sont -CH₂CH₂-, -CH₂CH₂CH₂- ou -CH₂CH₂CH₂CH₂-, R₈ est (C₁-C₂₁)-alkyle saturé ou mono-insaturé, éventuellement substitué, dans lequel les jusqu'à cinq substituants identiques ou différents sont indépendamment choisi parmi -OH, (C₁-C₆)-alkyle, (C₁-C₆)-alkoxy (C₁-C₆)-alkyle, (C₁-C₆)-alkanoyle, amino, halogèno, cyano, azido, oxo, mercapto, et nitro ; et X₂ est un groupe partant ou -OH, sous réserve que lorsque R₆ et R₇ sont -CH₂CH₃ et R₈ est -CH₃, alors X₂ n'est pas (a) -OH, (b) -O-C(=O)-C(CH₃)₃ ou (c) -O-SO₂-CH₃.

26. Composé selon la revendication 25, dans laquelle R₈ est (C₃-C₂₁)-alkyle saturé ou mono-insaturé, éventuellement substitué, dans lequel les jusqu'à cinq substituants identiques ou différents sont indépendamment choisi parmi -OH, (C₁-C₆)-alkyle, (C₁-C₆)-alkoxy (C₁-C₆)-alkyle, (C₁-C₆)-alkanoyle, amino, halogèno, cyano, azido, oxo, mercapto, et nitro.

27. Composé selon les revendications 25 ou 26, dans laquelle R₆ et R₇ sont -CH₃ ou -CH₂CH₃ ou R₆ et R₇ pris ensemble sont -CH₂CH₂-, -CH₂CH₂CH₂- ou -CH₂CH₂CH₂CH₂-, R₈ est -(CH₂)₁₆CH_{3,} et X₂ est un halogène, ou un groupe partant sulfonate, ou -OH.

28. Composé selon la revendication 27, dans laquelle R₆ et R₇ sont -CH₂CH₃, R₈ est -(CH₂)₁₆CH₃ et X₂ est tosylate ou -OH.

29. Composé selon la revendication 25, dans laquelle le composé est le (2S)-2-acétoxyméthyl-4,4-diéthoxybutyl toluènesulfonate.

30. Procédé de préparation d'un composé selon l'une quelconque des revendications 25 à 29, le procédé comprenant les étapes de :
a) réaction d'un composé de formule : 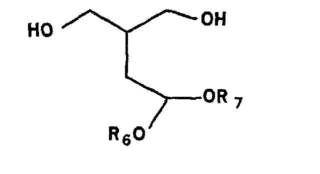 dans laquelle R₆ et R₇ sont tels que définis ci-dessus, avec CH₂=CH-OC(O)R₈, en présence d'une lipase pour fournir un ester de formule : 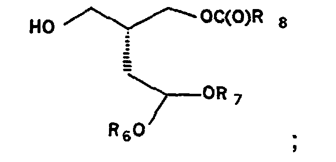 et éventuellement, lorsqu'on souhaite que X₂ soit un groupe partant,
b) conversion du groupe hydroxyle du produit de l'étape a) en un groupe partant.

31. Composé de formule : 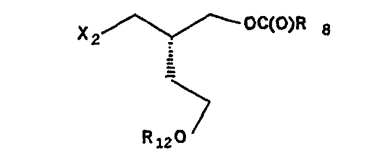 dans laquelle R₈ est (C₁-C₂₁)-alkyle saturé ou mono-insaturé, éventuellement substitué, dans lequel les jusqu'à cinq substituants identiques ou différents sont indépendamment choisis parmi -OH, -(C₁-C₆)-alkyle, (C₁-C₆)-alkoxy, (C₁-C₆)-alkoxy (C₁-C₆)-alkyle, (C₁-C₆)-alkanoyle, amino, halogèno, cyano, azido, oxo, mercapto, et nitro ; et R₁₂ est -CH(Ph)₂, -C(Ph)₃ ou Si(tert-Bu)(CH₃)_{2,} où Ph est phényle et X₂ est un groupe partant ou -OH, sous réserve que lorsque R₈ est méthyle et X₂ est -OH, alors R₁₂ n'est pas -Si(tert-butyl)(CH₃)₂.

32. Composé selon la revendication 31, dans laquelle R₈ est -(CH₂)₁₆CH₃ et X₂ est -OH.

33. Composé selon la revendication 31, dans laquelle R₈ est -(CH₂)₁₆CH₃ et X₂ est un halogène ou un groupe partant sulfonate.

34. Composé selon la revendication 31, dans laquelle R₈ est -(CH₂)₁₆CH₃ et X₂ est tosylate.

35. Composé selon la revendication 31, dans laquelle R₈ est -CH₃ et X₂ est -OH.

36. Composé selon la revendication 31, dans laquelle R₈ est -CH₃ et X₂ est un halogène ou un groupe partant sulfonate.

37. Composé selon la revendication 31, dans laquelle R₈ est -CH₃ et X₂ est tosylate.

38. Procédé de préparation d'un composé selon l'une quelconque des revendications 31 à 37, dans lesquelles R₈ est (C₃-C₂₁)-alkyle saturé ou mono-insaturé, éventuellement substitué, dans lesquelles le procédé comprend les étapes de :
a) réaction d'un compose de formule : 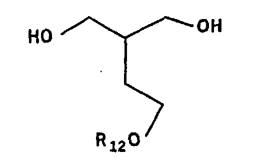 dans laquelle R₁₂ est tel que défini ci-dessus, avec CH₂=CH-OC(O)R₈, dans laquelle R₈ est tel que défini ci-dessus, en présence d'une lipase pour fournir un ester de formule : 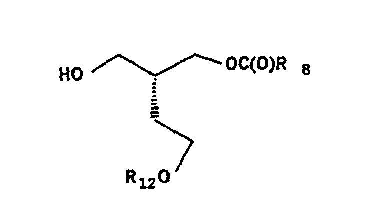 dans laquelle R₈ et R₁₂ sont tels que définis ci-dessus ; et éventuellement, lorsque l'on souhaite que X₂ soit un groupe partant,
b) conversion du groupe hydroxyle du produit de l'étape a) en un groupe partant.

39. Composé de formule : 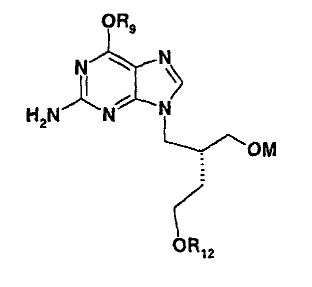 dans laquelle R₉ est -H ou un groupe protecteur de la fonction alcool choisi parmi les éthers méthyliques, les éthers de tétrahydropyranyle, les éthers éthyliques substitués, les éthers de silyle, et les esters d'acide carboxylique, dans laquelle lorsque R₉ est -H, la forme tautomère céto correspondante est incluse ; dans laquelle R₁₂ est -CH(Ph)₂, -C(Ph)₃, ou -Si(tert-Bu)(CH₃)₂, dans laquelle Ph est phényle, M est -H ou -C(O)R₁₀, R₁₀ étant un groupe (C₃-C₂₁)-alkyle saturé ou mono-insaturé, éventuellement substitué, dans lequel les jusqu'à cinq substituants identiques ou différents sont indépendamment choisis parmi -OH, (C₁-C₆)-alkyle, (C₁-C₆)-alkoxy, (C₁-C₆)-alkoxy (C₁-C₆)-alkyle, (C₁-C₆)-alkanoyle, amino, halogèno, cyano, azido, oxo, mercapto, et nitro, sous réserve que
(a) M est -H lorsque R₉ est un groupe protecteur de la fonction alcool ;
(b) R₁₂ n'est pas -C(Ph)₃ lorsque M et R₉ sont tous deux -H; et
(c) R₁₂ n'est pas -C(Ph)₃ lorsque M est -C(O)R₁₀ et R₉ est -H.

40. Composé selon la revendication 39, dans laquelle R₉ et M sont tous deux -H, R₉ est benzyle et M est -H, et/ou R₁₀ est -(CH₂)₁₆CH₃ et R₉ est -H.

41. Composé de formule : 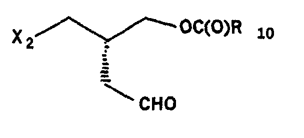 dans laquelle X₂ est un halogène ou un groupe partant sulfonate, R₁₀ est (C₃-C₂₁)-alkyle saturé or mono-insaturé, éventuellement substitué, dans lequel les jusqu'à cinq substituants identiques ou différents sont indépendamment choisis parmi -OH, (C₁-C₆)-alkyle, (C₁-C₆)-alkoxy, (C₁-C₆)-alkoxy (C₁-C₆)-alkyle, (C₁-C₆)-alkanoyle, amino, halogèno, cyano, azido, oxo, mercapto, et nitro, M est -CHO ou 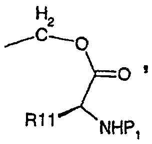 P₁ est un groupe N-protecteur, et R₁₁ est isopropyle ou isobutyle.

42. Composé selon la revendication 41, dans laquelle X₂ est p-toluènesulfonyloxy, R₁₀ est -(CH₂)₁₆CH₃, et L est -CHO ou -CH₂OC(O)CHR₁₁NHP₁, R₁₁ étant isopropyle et P₁ étant benzyloxycarbonyle.
